# EUROPEAN PATENT SPECIFICATION

(11) **EP 1 581 528 B1**
(45) Date of publication and mention of the grant of the patent: **01.08.2007**
(21) Application number: 03789563.8
(22) Date of filing: 24.12.2003
(51) Int. Cl.: C07D 417/04, C07D 417/14, C07D 487/04, A61K 31/427, A61P 35/00

(54) **PIPERIDINYL-THIAZOLE CARBOXYLIC ACID DERIVATIVES AS ANGIOGENESIS INHIBITORS**
PIPERIDINYL-THIAZOL-CARBONSÄURE-DERIVATE ALS ANGIOGENESIS INHIBITOREN
DERIVES D'ACIDE CARBOXYLIQUE PIPERIDINYL-THIAZOLE SERVANT D'INHIBITEURS DE L'ANGIOGENESE

(30) Priority: 24.12.2002 GB 0230162
(43) Date of publication of application: 05.10.2005
(73) Proprietor: Metris Therapeutics Limited, Reading RG6 1PT (GB)
(72) Inventor: KNOX, Peter, Reading RG6 1PT (GB); O'SULLIVAN, Michele, Reading RG6 1PT (GB); LENTFER, Heike, Reading RG6 1PT (GB)
(74) Representative: Goodfellow, Hugh Robin
(86) International application number: PCT/GB2003/005651
(87) International publication number: WO 2004/058750

(56) References cited:
- WO-A-95/33050
- WO-A-99/00356
- US-A- 3 966 748

## Description

### Technical Field

This invention relates to compounds that are useful in treating vascular endothelial growth factor (VEGF)-mediated disorders. In particular, this invention relates to compounds useful in treating endometriosis. The invention also relates to the use of these compounds and to pharmaceutical compositions comprising these compounds.

### Technical Background

Vasculogenesis and angiogenesis play important roles in a variety of physiological processes such as embryonic development, wound healing, organ regeneration and female reproductive processes. Unregulated angiogenesis occurs in a number of disease states. These include benign conditions such as endometriosis but also life-threatening conditions such as malignant tumours. The diverse disease states in which unregulated angiogenesis is present have been grouped together as angiogenic dependent or angiogenic associated diseases (Klagsburn & Soker, 1993, Current Biology 3(10):699-702; Folkman, 1991, J. Natl., Cancer Inst. 82:4-6; Weidner, et al., 1991, New Engl. J. Med. 324:1-5. Folkman & Shing, 1992, J. Biological Chem. 267(16):10931-34).

Several polypeptides with in vitro endothelial cell growth promoting activity have been identified but VEGF has been reported to be an endothelial cell specific mitogen (Ferrara & Henzel, 1989, Biochem. Biophys. Res. Comm. 161:851-858; Vaisman et al., 1990, J. Biol. Chem. 265:19461-19566).

VEGF is a family of dimeric glycoproteins that belong to the platelet derived growth factor (PDGF) superfamily of growth factors. In addition to VEGF-A, VEGF-B, VEGF-C and VEGF-D there is the so-called placental growth factor (P1GF). Some of the genes for these growth factors can be expressed as different isoforms. For example the VEGF-A gene is differentially spliced into a number of isoforms the most common messenger RNA's encoding polypeptides of 121, 165 and 189 amino acids. The compounds described in this invention are likely to have inhibitory activity against the VEGF family and possibly against other related families to a greater or lesser extent.

Thus the ability to inhibit the activity of VEGF and its stimulation of new blood vessel formation represents a selective pharmaceutical approach for a number of clinical conditions.

As mentioned above, one disease in which VEGF plays a role is endometriosis. Endometriosis is the name given to the disease resulting from the presence of endometrial cells outside of the uterine cavity. This disease affects women during their childbearing years with deleterious social, sexual and reproductive consequences. Endometriosis has been proposed as one of the most commonly-encountered diseases of gynaecology, with the incidence of endometriosis in the general population being estimated to be around 5%, although it is thought that at least 25% of women in their thirties and forties may have endometrical legions from this disease.

The development and maintenance of endometriosis involves the establishment and subsequent sustained growth of endometrial cells at ectopic sites, most commonly the pelvic peritoneum and ovaries, following retrograde menstruation (see Thomas & Prentice (1992) Repro. Med. Rev. (1): 21-36). Implantation of autologous non-malignant ectopic tissue is a unique phenomenon suggesting that an abnormal host response may be present in women who develop this disease. This theory is supported by the fact that only a minority of women will develop the disease in spite of the common occurrence of retrograde menstruation as a source of endometrial tissue.

There are many theories proposed for the origin of endometriosis and various cellular and biochemical constituents of the peritoneal fluid have been reported to play an important role in the pathogenesis of this disease. Alterations in multiple aspects of both humoral immunity and cell-mediated immunity have also been reported in suffering individuals.

The heritable aspects of endometriosis have been investigated in several studies (Moen & Magnus (1993) Acta Obstet. Gynecol. Scand., 72: 560-564; Kennedy et al, (1995) J. Assist. Repro. Gen., 12(1): 32-35; Malinak et al (1986) Am. J. Obstet. Gynecol., 137(3): 332-337; Treloar et al., (1999) Fertility Sterility 71(4) 701-710). On the basis of these studies, it has been hypothesised that endometriosis has in part a genetic basis. However, the precise aetiology of this disease still remains unknown.

The growth and development of endometrial tissue appears to depend on the presence of oestrogen. Drugs have thus been developed that reduce the body's oestrogen content in order to reduce the growth of endometrial implants at ectopic sites. Strategies include mimicking pregnancy, preventing ovulation using contraceptive agents, blocking the action of progesterone and mimicking the menopause. Although some of these drugs have proved successful, many cause unpleasant side-effects including post-menopausal like side effects and infertility, which mean that treatment must be discontinued to avoid the side-effects becoming permanent. Furthermore, all drugs described to date act by relieving the symptoms of the disease and are not in any sense curative. This makes a patient permanently dependent on the drug if the symptoms of disease are to be kept at bay.

Presently, the only treatment of endometriosis that is effective in the long term involves surgery. Therefore, there remains a great need for the discovery of agents with effective prophylactic, therapeutic and diagnostic value against endometriosis.

VEGF, also known as vascular permeability factor is secreted by tumours (Dvorak, H. et al., (1979) J. Immunol., 122, 166-174; it is a multi-functional cytokine that promotes the formation of blood vessels (angiogenesis). The growth of most tumours is dependent on the development of an adequate blood supply and therefore the prevention of angiogenesis by the inhibition of VEGF provides a potential strategy for the development of anti-cancer pharmaceuticals.

The expression of VEGF correlates with poor prognosis (Tabone, M. D. et al., (2001) Clin. Cancer Res., 7, 538-543) and has been detected in renal cell carcinoma (Slaton, J. W. et al., (2001) Am. J. Path., 158, 735-743), mammary carcinoma (Adams, J. et al., (2000) Cancer Res., 60, 2898-2905), head and neck squamous cell carcinoma (Minet, H. et al., (2000) Br. J. Cancer, 83, 775-781), bladder cancer (Inoue, K. et al., (2000) Clin. Cancer Res., 6, 4866-4873), oesophageal carcinoma (Shih, C. H. et al., (2000) Clin. Cancer Res., 6, 1161-1168),osteosarcoma (Kaya, M. et al., (2000) Clin. Cancer Res., 6,572-577), colonic carcinoma (Cascinu, S. et al., (2000) Clin. Cancer Res., 6, 2803-2807), ovarian carcinoma (Shen, G. H. et al., (2000) Br. J. Cancer, 83, 196-203), carcinoma of the cervix (Loncaster, J. A. et al., (2000) Br. J. Cancer, 83, 620-625), soft tissue sarcomas ( Yudoh, K. et al., (2001) Br. J. Cancer, 84, 1610-1615), astrocytoma (Abdulrauf, S. I. et al., (1998) J. Neurosurg., 88, 513-520) and prostate carcinoma (Borre, M. et al., (2000) Clin. Cancer Res., 6, 1882-1890). Inhibition of VEGF and thereby reducing the ability of the tumour to become vascularized, either alone or in combination with other treatments such as chemotherapy or radiotherapy my therefore have clinical utility in these and other human and animal tumours.

A number of non-oncological clinical indications involve abnormally increased angiogenesis and the inventions described herein may be the basis for therapeutic intervention. Macular degeneration and retinopathy can occur as a result of the ageing process or occur as a result of other diseases in particular diabetes. High levels of VEGF have been implicated in these conditions (Funatsu, H. et al., (2002) J. Cataract Refract. Surg. 28, 1355; Noma, H. et al., (2002) Arch. Ophthalmol. 120, 1075-80). It has been suggested that inhibition of VEGF may be useful (Aiello, L.P. (1997) Ophthalmic Res., 29,354-62) and in particular may prevent the oedema that occurs at the early stages of diabetic retinopathy (Lu, M. et al., (2002) Ophthalmol. Clin. North Am., 15, 69-79). Diabetic nephropathy and neuropathy may have a common biochemical dysfunction to retinopathy (Tilton, R.G. (2002) Microsc. Res. Tech., 57, 390-407) and an anti-VEGF pharmaceutical approach may be appropriate.

Inhibition of VEGF may also be a suitable therapeutic strategy in atheroma (Blann, A.D., (2002) Clin. Sci. 102, 187-94) and in rheumatoid arthritis (Afuwape, A.O., (2002) Histol. Histopathol., 17, 961-72) and psoriasis (Creamer, D. et al., (2002) Arch. Dermatol., 138, 791-6); VEGF has been implicated in the pathogenesis of both conditions.

It is an object of the present invention to provide compounds that are useful in treating VEGF-related disorders.

### Summary of the Invention

According to a first embodiment of the present invention there is provided a compound of formula (I) or formula (II): 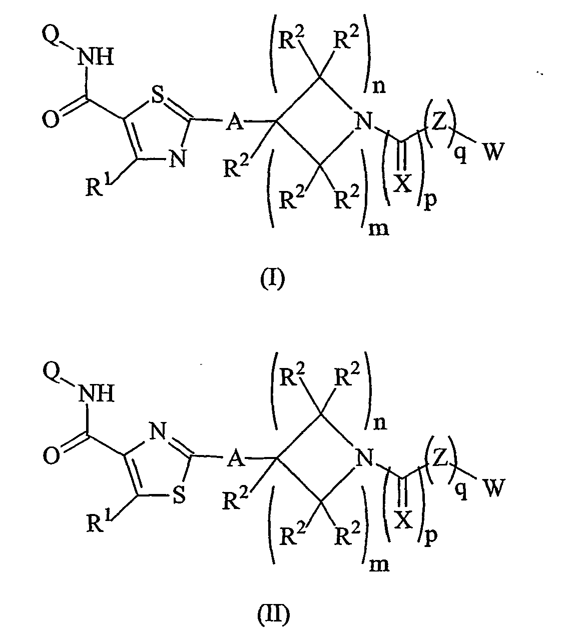 wherein:
Q is an optionally substituted alkyl, alkenyl, cycloalkyl, aryl, aralkyl, alkoxy, aryloxy, aralkoxy, alkylthio, aralkylthio, amino, alkylamino, dialkylamino, carboxyl, carboxylalkyl, esterified carboxyl, alkylsulfoxyl, alkylsulfonyl, nitro, carbonitrile, carbo-alkoxy, carbo-aryloxy, or heterocyclic group;
A is a single bond or alkylene;
X is O or S;
Z is O, S or NR³;
p is 0 or 1
q is 0 or 1;
n is an integer from 0 to 10;
m is an integer from 0 to 10;
W is an optionally substituted alkyl, alkenyl, cycloalkyl, aryl, aralkyl, alkoxy, aryloxy, aralkoxy, alkylthio, aralkylthio, amino, alkylamino, dialkylamino, carboxyl, carboxylalkyl, esterified carboxyl, alkylsulfoxyl, alkylsulfonyl, nitro, carbonitrile, carbo-alkoxy, carbo-aryloxy, or heterocyclic group;
R¹ is H or alkyl;
R² is independently H or alkyl; and
R³ is H or alkyl;
or a pharmaceutically acceptable derivative thereof

The term "pharmaceutically acceptable derivative" as used herein, means any pharmaceutically acceptable salt, addition compound, or any other compound which upon administration to a recipient is capable of providing, whether directly or indirectly, a compound of the invention or a pharmaceutically acceptable metabolite.

The term "pharmaceutically acceptable metabolite" as used herein, means a metabolite or residue of a compound of the invention which gives rise to a biological activity exhibited by the compounds of the invention.

The term "pharmaceutically acceptable salt", as used herein, refers to a salt prepared from pharmaceutically acceptable non-toxic acids or bases including inorganic or organic acids and bases.

Examples of such inorganic acids are hydrochloric, hydrobromic, hydroiodic, sulfuric, and phosphoric acids. Appropriate organic acids may be selected, for example, from aliphatic, aromatic, carboxylic and sulfonic classes of organic acids, examples of which are formic, acetic, propionic, succinic, glycolic, glucuronic, maleic, furoic, glutamic, benzoic, anthranilic, salicylic, phenylacetic, mandelic, embonic (pamoic), methanesulfonic, ethanesulfonic, pantothenic, benzenesulfonic, stearic, sulfanilic, algenic, and galacturonic. Examples of such inorganic bases include metallic salts made from aluminium, calcium, lithium, magnesium, potassium, sodium, and zinc. Appropriate organic bases may be selected, for example, from N,N-dibenzylethylenediamine, chloroprocaine, choline, diethanolamine, ethylenediamine, meglumaine (N-methylglucamine), and procaine.

As used herein, the term "alkyl" means a branched or unbranched, cyclic or acyclic, saturated or unsaturated (e.g. alkenyl or alkynyl) hydrocarbyl radical. Where cyclic, the alkyl group is C₃ to C₁₂, more preferably C₅ to C₁₀, more preferably C₅, C₆ or C₇. Where acyclic, the alkyl group is C₁ to C₁₀, more preferably C₁ to C₆, more preferably methyl, ethyl, propyl (n-propyl or isopropyl), butyl (n-butyl, isobutyl or tertiary-butyl) or pentyl (including n-pentyl and iso-pentyl), more preferably methyl. It will be appreciated therefore that the term "alkyl" as used herein includes alkyl (branched or unbranched), alkenyl (branched or unbranched), alkynyl (branched or unbranched), cycloalkyl, cycloalkenyl and cycloalkynyl.

Saturated hydrocarbyl radicals are generally preferred.

As used herein, the term "lower alkyl" means a branched or unbranched, cyclic or acyclic, saturated or unsaturated (e.g. alkenyl or alkynyl) hydrocarbyl radical, wherein a cyclic lower alkyl group is C₅, C₆ or C₇, and wherein an acyclic lower alkyl group is C₁ to C₆, that is, methyl, ethyl, propyl (n-propyl or isopropyl) or butyl (n-butyl, isobutyl or tertiary-butyl), more preferably methyl.

An alkyl group may be substituted or unsubstituted. Where substituted, there will generally be 1 to 3 substituents present, preferably 1 or 2 substituents. Substituents may include halogen atoms and halomethyl groups such as CF₃ and CCl₃; oxygen containing groups such as oxo, hydroxy, carboxy, carboxyalkyl, alkoxy, alkoxy, alkoyl, alkoyloxy, aryloxy, aryloyl and aryloyloxy; nitrogen containing groups such as amino, amido, alkylamino, dialkylamino, cyano, azide, nitrato and nitro; sulphur containing groups such as thiol, alkylthiol, sulphonyl and sulphoxide; heterocyclic groups containing one or more, heteroatom, such as thienyl, furanyl, pyrrolyl, imidazolyl, pyrazolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolyl, isoquinolyl, naphthridinyl, cinnolinyl, quinazolinyl, pyridopyridyl, benzoxazinyl, quinoxadinyl, chromenyl, chromanyl, isochromanyl, carbolinyl, oxadiazolyl, thiadiazolyl, triazolyl, tetrazolyl and pyrimidinyl; alkyl groups, which may themselves be substituted; and aryl groups, which may themselves be substituted, such as phenyl and substituted phenyl.

As used herein, the term "alkylene" means a branched or unbranched, cyclic or acyclic, saturated or unsaturated (e.g. alkenylene or alkynylene) hydrocarbylene radical. Where cyclic, the alkylene group is C₃ to C₁₂, more preferably C₅ to C₁₀, more preferably C₅ to C₇. Where acyclic, the alkylene group is C₁ to C₁₆, more preferably C₁ to C₄, more preferably methylene.

An alkylene group may be substituted or unsubstituted, preferably unsubstituted. Where substituted, there will generally be 1 to 3 substituents present, preferably 1 substituent. Substituents may include halogen atoms and halomethyl groups such as CF₃ and CCl₃; oxygen containing groups such as oxo, hydroxy, carboxy, carboxyalkyl, alkoxy, alkoxy, alkoyl, alkoyloxy, aryloxy, aryloyl and aryloyloxy; nitrogen containing groups such as amino, amido, alkylamino, dialkylamino, cyano, azide, nitrato and nitro; sulphur containing groups such as thiol, alkylthiol, sulphonyl and sulphoxide; heterocyclic groups containing one or more, preferably one, heteroatom, such as thienyl, furanyl, pyrrolyl, imidazolyl, pyrazolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolyl, isoquinolyl, naphthridinyl, cinnolinyl, quinazolinyl, pyridopyridyl, benzoxazinyl, quinoxadinyl, chromenyl, chromanyl, isochromanyl and carbolinyl; alkyl groups, which may themselves be substituted; and aryl groups, which may themselves be substituted, such as phenyl and substituted phenyl.

As used herein, the term "aryl" means C₆₋₁₂ (e.g. C₆₋₁₀) aryl groups and is a cyclic or bicyclic aromatic group, such as phenyl or naphthyl. C₆₋₁₂ (e.g. C₆₋₁₀) aryl groups are preferred. An aryl group may be substituted or unsubstituted, preferably unsubstituted. Where substituted, there will generally be 1 to 3 substituents present, preferably 1 substituent. Substituents may include halogen atoms and halomethyl groups such as CF₃ and CCl₃; oxygen containing groups such as oxo, hydroxy, carboxy, carboxyalkyl, alkoxy, alkoxy, alkoyl, alkoyloxy, aryloxy, aryloyl and aryloyloxy; nitrogen containing groups such as amino, amido, alkylamino, dialkylamino, cyano, azide, nitrato and nitro; sulphur containing groups such as thiol, alkylthiol, sulphonyl and sulphoxide; heterocyclic groups containing one or more, preferably one, heteroatom, such as thienyl, furanyl, pyrrolyl, imidazolyl, pyrazolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolyl, isoquinolyl, naphthridinyl, cinnolinyl, quinazolinyl, pyridopyridyl, benzoxazinyl, quinoxadinyl, chromenyl, chromanyl, isochromanyl and carbolinyl; alkyl groups, which may themselves be substituted; and aryl groups, which may themselves be substituted, such as phenyl and substituted phenyl.

As used herein, the term "heterocyclic" means a saturated or unsaturated cyclic or bicyclic group containing one or more heteroatoms, such as thienyl, furanyl, pyrrolyl, imidazolyl, pyrazolyl, thiazolyl, oxazolyl, isoxazolyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolinyl, isoquinolinyl, naphthridinyl, cinnolinyl, quinazolinyl, pyridopyridyl, benzoxazinyl, quinoxadinyl, chromenyl, chromanyl, isochromanyl, carbolinyl, oxadiazolyl, thiadiazolyl, triazolyl, tetrazolyl and pyrimidinyl.

The term "heterocyclic group" also includes groups derived from: 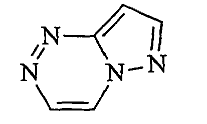 preferably 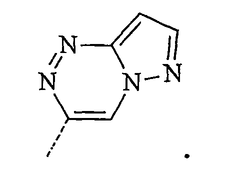 Heterocyclic groups contain from 5 to 12 (e.g. 5 to 10) atoms. Heterocyclic groups contain 1, 2, 3 or 4 heteroatoms. Heteroatoms are N, O and S. S.

A heterocyclic group may be substituted or unsubstituted, preferably unsubstituted. Where substituted, there will generally be 1 to 3 substituents present, preferably 1 substituent. Substituents may include halogen atoms and halomethyl groups such as CF₃ and CCl₃; oxygen containing groups such as oxo, hydroxy, carboxy, carboxyalkyl, alkoxy, alkoxy, alkoyl, alkoyloxy, aryloxy, aryloyl and aryloyloxy; nitrogen containing groups such as amino, amido, alkylamino, dialkylamino, cyano, azide, nitrato and nitro; sulphur containing groups such as thiol, alkylthiol, sulphonyl and sulphoxide; heterocyclic groups containing one or more, preferably one, heteroatom, such as thienyl, furanyl, pyrrolyl, imidazolyl, pyrazolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolyl, isoquinolyl, naphthridinyl, cinnolinyl, quinazolinyl, pyridopyridyl, benzoxazinyl, quinoxadinyl, chromenyl, chromanyl, isochromanyl and carbolinyl; alkyl groups, which may themselves be substituted; and aryl groups, which may themselves be substituted, such as phenyl and substituted phenyl.

When a heterocyclic group, which is itself a substituent, is substituted, it is preferably substituted with lower alkyl, more preferably methyl.

As used herein, the term "aralkyl" means aryl-alkyl- (e.g. benzyl).

As used herein, the term "alkoxy" means alkyl-O-. As used herein, the term "lower alkoxy" means loweralkyl-O-. As used herein, the term "aryloxy" means aryl-O-. As used herein, the term "aralkoxy" means aralkyl-O-,

As used herein, substituents which are nitrogen containing groups include -C(O)-NH₂, -C(O)-NHR⁴ and -C(O)-NR⁴₂, where R⁴ is independently an optionally substituted alkyl or aryl (preferably alkyl).

As used herein, substituents which are sulphur containing groups include -S(O)₂-H, -S(O)₂-R⁴, -S(O)-H and -S(O)-R⁴, where R⁴ is an optionally substituted alkyl or aryl (preferably alkyl).

As used herein, the term "halogen" means a fluorine, chlorine, bromine or iodine radical, preferably a fluorine or chlorine radical.

Compounds of the invention of formula (II) are preferred.

Preferably, A is a single bond.

Preferably, X is O. Alternatively, it is preferred that X is S and Z is N.

Preferably, X is O. Alternatively, it is preferred that X is S and Z is NR³.

Preferably, R³ is H.

Preferably, p = 1.

Preferably, q = 0.

Preferably, the sum n +m is an integer from 2 to 10, more preferably 2 to 6, more preferably 2 to 4, more preferably 3 or 4, most preferably 4.

Preferably, n is from 0 to 3, preferably 2.

Preferably, m is from 0 to 3, preferably 2.

Preferably, n = 2 and m = 2.

Preferably, R¹ is H.

Preferably, each R² is H.

The substituents Q and W may be substituted or unsubstituted. Where substituted, there will generally be 1 to 3 substituents present, preferably 1 or 2 substituents. Substituents may include halogen atoms and halomethyl groups such as CF₃ and CCl₃; oxygen containing groups such as oxo, hydroxy, carboxy, carboxyalkyl, alkoxy, alkoxy, alkoyl, alkoyloxy, aryloxy, aryloyl and aryloyloxy; nitrogen containing groups such as amino, amido, alkylamino, dialkylamino, cyano, azide, nitrato and nitro; sulphur containing groups such as thiol, alkylthiol, sulphonyl and sulphoxide; heterocyclic groups containing one or more, heteroatom, such as thienyl, furanyl, pyrrolyl, imidazolyl, pyrazolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolyl, isoquinolyl, naphthridinyl, cinnolinyl, quinazolinyl, pyridopyridyl, benzoxazinyl, quinoxadinyl, chromenyl, chromanyl, isochromanyl, carbolinyl, oxadiazolyl, thiadiazolyl, triazolyl, tetrazolyl and pyrimidinyl; alkyl groups, which may themselves be substituted; and aryl groups, which may themselves be substituted, such as phenyl and substituted phenyl.

Preferably, Q is an optionally substituted alkyl, alkenyl, cycloalkyl, aryl, aralkyl, carboxyl, carboxylalkyl, esterified carboxyl, alkylsulfonyl or heterocyclic group.

More preferably, Q comprises an optionally substituted aryl or heterocyclic group. Still more preferably, Q is an optionally substituted aryl or heterocyclic group. A preferred aryl group is phenyl. A preferred heterocyclic group is an unsaturated heterocyclic group, more preferably a monocyclic unsaturated heterocyclic group.

When substituted, Q is preferably independently substituted by one or more (e.g. 1, 2 or 3) of: halogen; trihalomethyl; -NO₂; -CN; -Y-C(=Y)-R⁵; -C(=Y)-R⁵; -C(=Y)-Y-R⁵; -Y-C(=Y)-Y-R⁵; -SOR⁵; -S(=O)₂R⁵; -Y-S(=O)OR⁵; -Y-S(=O)₂R⁵; -S(=O)₂-YR⁵; -Y-S(=O)₂-YR⁵; -R⁵; -YR⁵; or alkyl (preferably methyl) substituted with one or more (e.g. 1, 2 or 3, preferably 1) of halogen, trihalomethyl, -NO₂, -CN, -Y-C(=Y)-R⁵, -C(=Y)-R⁵, -C(=Y)-Y-R⁵, -Y-C(=Y)-Y-R⁵, -SOR⁵, -S(=O)₂R⁵, -Y-S(=O)OR⁵, -Y-S(=O)₂R⁵, -S(=O)₂-YR⁵, -Y-S(=O)₂-YR⁵ or -YR⁵. Y is independently O, S or NR⁵, and R⁵ is independently H or an optionally substituted alkyl, aryl or heterocyclic group (preferably H or alkyl). More preferred substituents are: halogen; trihalomethyl; -NO₂; -CN; -CO₂H; -CO₂R⁵; -C(=O)H; -C(=O)R⁵; -OC(=O)R⁵; -OC(=O)OR⁵; -C(=O)NH₂; -C(=O)NR⁵₂; -N(R⁵)C(=O)R⁵; -N(R⁵)C(=O)OR⁵; -OC(=O)NR⁵₂; -N(R⁵)C(=O)NR⁵₂; -C(=S)NH₂; -C(=S)NR⁵₂; -N(R⁵)C(=S)R⁵; -N(R⁵)C(=S)NR⁵₂; -C(=NH)NH₂; -C(=NR⁵)NR⁵₂; -N(R⁵)C(=NR⁵)R⁵; -N(R⁵)C(=NR⁵)NR⁵₂; -SOR⁵; -S(=O)₂R⁵; -S(=O)₂OH; -S(=O)₂OR⁵; -S(=O)₂NR⁵₂; -N(R⁵)SO₂R⁵; -N(R⁵)SO₂NR⁵₂; -NR⁵₂; -R⁵; -YR⁵; or alkyl (preferably methyl) substituted with one or more (e.g. 1, 2 or 3, preferably 1) of halogen, trihalomethyl, -NO₂, -CN, -CO₂H, -CO₂R⁵, -C(=O)H, -C(=O)R⁵, -OC(=O)R⁵, -OC(=O)OR⁵, -C(=O)NH₂, -C(=O)NR⁵₂, -N(R⁵)C(=O)R⁵, -N(R⁵)C(=O)OR⁵, -OC(=O)NR⁵₂, -N(R⁵)C=O)NR⁵₂, -C(=S)NH₂, -C(=S)NR⁵₂, -N(R⁵)C(=S)R⁵, -N(R⁵)C(=S)NR⁵₂, -C(=NH)NH₂, -C(=NR⁵)NR⁵₂, -N(R⁵)C(=NR⁵)R⁵, -N(R⁵)C(=NR⁵)NR⁵₂, -SOR⁵, -S(=O)₂R⁵, -S(=O)₂OH, -S(=O)₂OR⁵, -S(=O)₂NR⁵₂, N(R⁵)SO₂R⁵, -N(R⁵)SO₂NR⁵₂, -NR⁵₂ or -YR⁵. Still more preferred substituents are: halogen; -CN; -CO₂H; -CO₂R⁵; -C(=O)R⁵; -C(=O)NH₂; -C(=O)NR⁵₂; -N(R⁵)C(=O)R⁵; -C(=S)NH₂; -C(=S)NR⁵₂; -C(=NH)NH₂; -C(=NR⁵)NR⁵₂; -S(=O)₂R⁵; -NR⁵₂; -R⁵; -YR⁵ or alkyl (preferably methyl) substituted with -C(=O)NR⁵₂. Still more preferred substituents are -Cl, -OMe, -C(=O)NH₂ and -CH₂-C(=O)NH₂.

When Q is substituted by -R⁵, it is preferred that R⁵ is an optionally substituted heterocyclic group, preferably an unsaturated heterocyclic group, preferably a monocyclic unsaturated heterocyclic group (e.g. oxazolyl, tetrazolyl or oxazolyl substituted with lower alkyl, e.g. methyl).

Preferably Q is a heterocyclic group, optionally substituted with 1, 2 or 3 substituents, preferably 2 substituents. When Q is a heterocyclic group, it is preferably thienyl (e.g. 2-thienyl) or furanyl (e.g. 2-furanyl).

Preferably, Q is a phenyl group optionally substituted with 1, 2 or 3 substituents, preferably 2 substituents. Preferably Q is a phenyl group having at least one substituent selected from alkoxy, amide, carboxy, carboxylalkyl, alkoxy, cyano, halogen and a heterocyclic group.

Preferably Q is a phenyl group substituted with at least one group selected from methoxy, cyano, chlorine, fluorine, oxazolyl, tetrazolyl, oxazolyl substituted with lower alkyl, -C(O)NH₂, -C(O)NHR, -C(O)NR₂, -C(S)NH₂ and -NHC(O)R, where R is lower alkyl, preferably methyl.

Q may be thiophenyl. Preferably, Q is thiophenyl substituted with a methoxy, cyano, chlorine, -C(O)NH₂, -C(O)NHR, -C(O)NR₂, -C(S)NH₂ or -NHC(O)R group, where R is lower alkyl, preferably methyl.

Q may be furanyl. Preferably, Q is furanyl substituted with a methoxy, cyano, chlorine, -C(O)NH₂, -C(O)NHR, -C(O)NR₂, -C(S)NH₂ or -NHC(O)R group, where R is lower alkyl, preferably methyl.

Q may be thienyl. Preferably, Q is thienyl optionally substituted with a methoxy, cyano, chlorine, -C(O)NH₂, -C(O)NHR, -C(O)NR₂ -C(S)NH₂ or -NHC(O)R group, where R is lower alkyl, preferably methyl.

Preferably Q is a radical selected from the radicals set out in Table 1. It will be understood that the linking nitrogen atom shown (-NH---) does not form part of the radical Q.

**Table 1**

| **Q** | **Radical** |
|---|---|
| Q¹ | 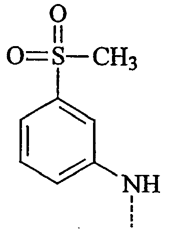 |
| Q² | 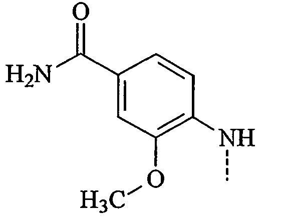 |
| Q³ | 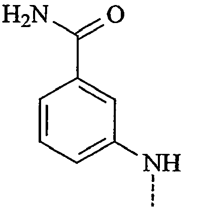 |
| Q⁴ | 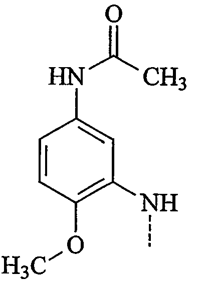 |
| Q⁵ | 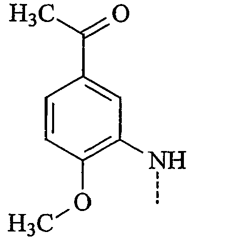 |
| Q⁶ | 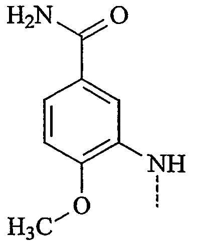 |
| Q⁷ | 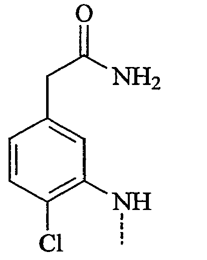 |
| Q⁸ | 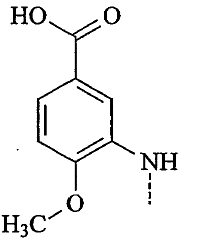 |
| Q⁹ | 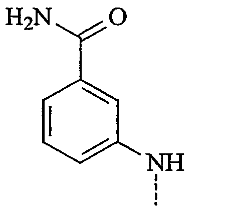 |
| Q¹⁰ | 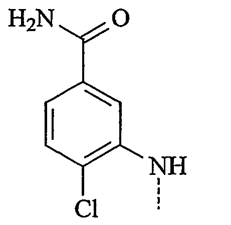 |
| Q¹¹ | 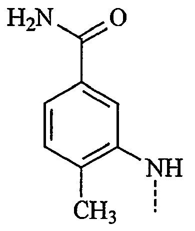 |
| Q¹² | 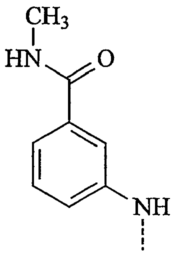 |
| Q¹³ | 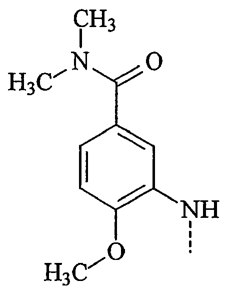 |
| Q¹⁴ | 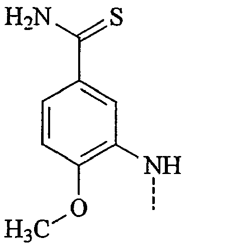 |
| Q¹⁵ | 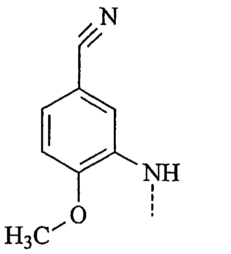 |
| Q¹⁶ | 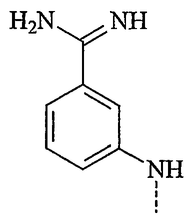 |
| Q¹⁷ | 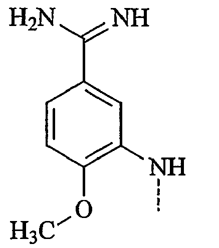 |
| Q¹⁸ | 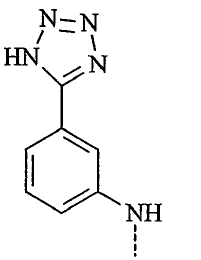 |
| Q¹⁹ | 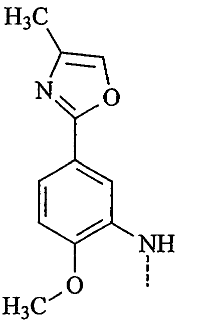 |
| Q²⁰ | 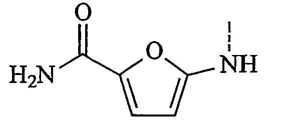 |
| Q²¹ | 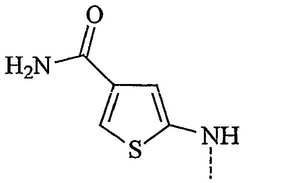 |
| Q²² | 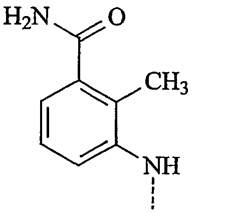 |
| Q²³ | 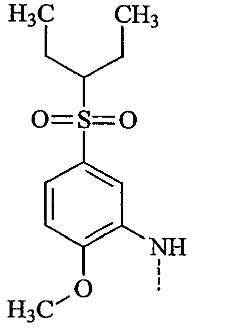 |
| Q²⁴ | 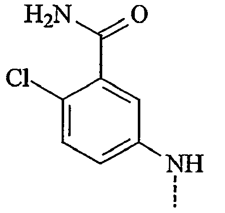 |
| Q²⁵ | 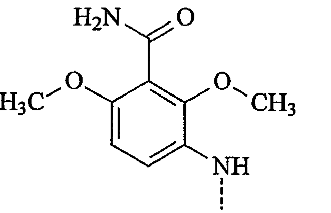 |
| Q²⁶ | 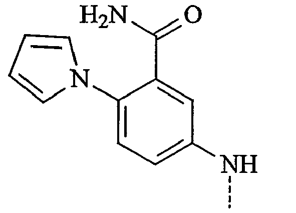 |

Preferred Q are Q₆, Q₇ and Q₁₀.

Preferably Q is 5-carbamoyl-2-methoxy-phenyl.

Preferably, W is an optionally substituted alkyl, alkenyl, cycloalkyl, aryl, aralkyl, alkoxy, aryloxy, aralkoxy, alkylthio, aralkylthio, carboxyl, carboxylalkyl, esterified carboxyl, alkylsulfonyl, carbo-alkoxy, carbo-aryloxy or heterocyclic group. More preferably, W is an optionally substituted alkyl, alkenyl, alkynyl, aryl or heterocyclic group. A preferred aryl group is phenyl. A preferred heterocyclic group is an unsaturated heterocyclic group, more preferably a monocyclic unsaturated heterocyclic group.

Where W is a substituted alkyl, alkenyl or alkynyl group, it is preferably substituted with an optionally substituted alkyl, aryl, heterocyclic, -Y¹-alkyl, -Y¹-aryl or -Y¹-(heterocyclic) group, where Y¹ is O, S or NR⁶ (preferably O or S) and R⁶ is independently H or alkyl. Where W is a substituted alkyl group, it is more preferably substituted with an optionally substituted aryl (e.g. phenyl), heterocyclic or -Y¹ -(heterocyclic) group.

In one embodiment, W is preferably a benzyl group optionally substituted on the phenyl ring.

Alternatively, where W is a substituted alkyl, alkenyl or alkynyl group, it may be substituted with -(O-alkylene)ₐ-O-alkyl (preferably -(O-ethylene)ₐ-O-alkyl or -(O-propylene)ₐ-O-alkyl), where a is from 1 to 20 (preferably 1 to 10, preferably 1 to 5, more preferably 2).

Where substituted, W is preferably independently substituted by one or more (e.g. 1, 2 or 3) of: halogen; trihalomethyl; -NO₂; -CN; -Y-C(=Y)-R⁵; -C(=Y)-R⁵; -C(=Y)-Y-R⁵; -Y-C(=Y)-Y-R⁵; -SOR⁵; -S(=O)₂R⁵; -Y-S(=O)OR⁵; -Y-S(=O)₂R⁵; -S(=O)₂-YR⁵; -Y-S(=O)₂-YR⁵; -R⁵; -YR⁵; or alkyl (preferably methyl) substituted with one or more (e.g. 1, 2 or 3, preferably 1) of halogen, trihalomethyl, -NO₂, -CN, -Y-C(=Y)-R⁵, -C(=Y)-R⁵, -C(=Y)-Y-R⁵, -Y-C(=Y)-Y-R⁵, -SOR⁵, -S(=O)₂R⁵, -Y-S(=O)OR⁵, -Y-S(=O)₂R⁵, -S(=O)₂-YR⁵, -Y-S(=O)₂-YR⁵ or -YR⁵. Y is independently O, S or NR⁵, and R⁵ is independently H or an optionally substituted alkyl, aryl or heterocyclic group (preferably H or alkyl). More preferred substituents are halogen, trihalomethyl, -NO₂, -CN, -CO₂H, -CO₂R⁵, -C(=O)H, -C(=O)R⁵, -OC(=O)R⁵, -OC(=O)OR⁵, -C(=O)NH₂, -C(=O)NR⁵₂, -N(R⁵)C(=O)R⁵, -N(R⁵)C(=O)OR⁵, -OC(=O)NR⁵₂, -N(R⁵)C(=O)NR⁵₂, -C(=S)NH₂, -C(=S)NR⁵₂, -N(R⁵)C(=S)R⁵, -N(R⁵)C(=S)NR⁵₂, -C(=NH)NH₂, -C(=NR⁵)NR⁵₂, -N(R⁵)C(=NR⁵)R⁵, -N(R⁵)C(=NR⁵)NR⁵₂, -SOR⁵, -S(=O)₂R⁵, -S(=O)₂OH, -S(=O)₂OR⁵, -S(=O)₂NR⁵₂, -N(R⁵)SO₂R⁵, -N(R⁵)SO₂NR⁵₂, -NR⁵₂, -R⁵ or -YR⁵. Still more preferred substituents are halogen, -CN, -CO₂H, -CO₂R⁵, -C(=O)R⁵, -C(=O)NH₂, -C(=O)NR⁵₂, -N(R⁵)C(=O)R⁵, -C(=S)NH₂, -C(=S)NR⁵₂, -C(=NH)NH₂, -C(=NR⁵)NR⁵₂, -S(=O)₂R⁵, -NR⁵₂, -R⁵ or -YR⁵.

Where W is a substituted alkyl, alkenyl or alkynyl group (preferably alkyl, preferably methyl) substituted with an optionally substituted alkyl (preferably cycloalkyl), aryl, heterocyclic, -Y¹-alkyl, -Y¹-aryl or -Y¹-(heterocyclic) group, the alkyl, aryl, heterocyclic, -Y¹-alkyl, -Y¹-aryl or -Y¹-(heterocyclic) substituent group may optionally be substituted by one or more (e.g. 1, 2 or 3) of: halogen; trihalomethyl; -NO₂; -CN; -Y-C(=Y)-R⁵; -C(=Y)-R⁵; -C(=Y)-Y-R⁵; -Y-C(=Y)-Y-R⁵; -SOR⁵; -S(=O)₂R⁵; -Y-S(=O)OR⁵; -Y-S(=O)₂R⁵; -S(=O)₂-YR⁵; -Y-S(=O)₂-YR⁵; -R⁵; -YR⁵; or alkyl (preferably methyl) substituted with one or more (e.g. 1, 2 or 3, preferably 1) of halogen, trihalomethyl, -NO₂, -CN, -Y-C(=Y)-R⁵, -C(=Y)-R⁵, -C(=Y)-Y-R⁵, -Y-C(=Y)-Y-R⁵, -SOR⁵, -S(=O)₂R⁵, -Y-S(=O)OR⁵, -Y-S(=O)₂R⁵, -S(=O)₂-YR⁵, -Y-S(=O)₂-YR⁵ or -YR⁵. Y is independently O, S or NR⁵, and R⁵ is independently H or an optionally substituted alkyl, aryl or heterocyclic group (preferably H or alkyl). More preferred substituents on the alkyl, aryl, heterocyclic, -Y¹-alkyl, -Y¹-aryl or -Y¹-(heterocyclic) substituent group are halogen, trihalomethyl, -NO₂, -CN, -CO₂H, -CO₂R⁵, -C(=O)H, -C(=O)R⁵, -OC(=O)R⁵, -OC(=O)OR⁵, -C(=O)NH₂, -C(=O)NR⁵₂, -N(R⁵)C(=O)R⁵, -N(R⁵)C(=O)OR⁵, -OC(=O)NR⁵₂, -N(R⁵)C(=O)NR⁵₂, -C(=S)NH₂, -C(=S)NR⁵₂, -N(R⁵)C(=S)R⁵, -N(R⁵)C(=S)NR⁵₂, -C(=NH)NH₂, -C(=NR⁵)NR⁵₂, -N(R⁵)C(=NR⁵)R⁵, -N(R⁵)C(=NR⁵)NR⁵₂, -SOR⁵, -S(=O)₂R⁵, -S(=O)₂OH, -S(=O)₂OR⁵, -S(=O)₂NR⁵₂, -N(R⁵)SO₂R⁵, -N(R⁵)SO₂NR⁵₂, -NR⁵₂, -R⁵ or -YR⁵. Still more preferred substituents are halogen, -CN, -CO₂H, -CO₂R⁵, -C(=O)R⁵, -C(=O)NH₂, -C(=O)NR⁵₂, -N(R⁵)C(=O)R⁵, -C(=S)NH₂, -C(=S)NR⁵₂, -C(=NH)NH₂, -C(=NR⁵)NR⁵₂, -S(=O)₂R⁵, -NR⁵₂, -R⁵ or -YR⁵.

Preferably, W comprises an optionally substituted aryl or heterocyclic group. More preferably, W is an optionally substituted aryl or heterocyclic group. A preferred aryl group is phenyl. A preferred heterocyclic group is an unsaturated heterocyclic group, more preferably a monocyclic unsaturated heterocyclic group.

Preferably W is a heterocyclic group, optionally substituted with 1, 2 or 3 substituents, preferably 2 substituents. Preferably W is an optionally substituted phenyl, oxazolyl, diazolyl, quinolinyl, benzofuranyl or pyrindinyl. Preferably, the substituents are independently selected from alkoxy, amide, carboxy, carboxylalkyl, alkoxy, cyano, halogen and a heterocyclic group, more preferably, methoxy, cyano, chlorine, oxazolyl, tetrazolyl, oxazolyl substituted with lower alkyl, -C(O)NH₂, -C(O)NHR, -C(O)NR₂, -C(S)NH₂ and -NHC(O)R, where R is lower alkyl, preferably methyl.

Alternatively, W is an alkyl, alkylene, alkylyne, alkyoxy or amine, carboxylalkyl, optionally substituted with a heterocyclic group. Preferably, the heterocyclic group is substituted with 1, 2 or 3 substituents independently selected from alkoxy, amide, carboxy, carboxylalkyl, alkoxy, cyano, halogen and a heterocyclic group. More preferably the substituents are methoxy, cyano, chlorine, oxazolyl, tetrazolyl, oxazolyl substituted with lower alkyl, -C(O)NH₂, -C(O)NHR, -C(O)NR₂, -C(S)NH₂ and -NHC(O)R, where R is lower alkyl, preferably methyl.

Preferably, the compound of the invention has the formula (III): 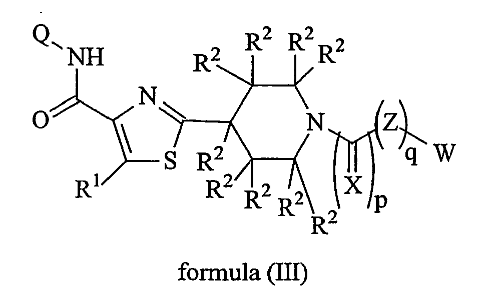 wherein Q, R¹, R², X, Z, W, p and q are as defined above.

In one embodiment of the invention, the compound of formula (I) is preferably:
*N*-[5-(aminocarbonyl)-2-methoxyphenyl]-2-{1-[(4,7-dimethylpyrazolo[5,1-*c*][1,2,4]triazin-3-yl)carbonyl]-4-piperidinyl}-1,3-thiazole-4-carboxamide;
*N*-[5-(aminocarbonyl)-2-methoxyphenyl]-2-[1-(1-benzofuran-2-ylcarbonyl)-4-piperidinyl]-1,3-thiazole-4-carboxamide;
*N*-[5-(aminocarbonyl)-2-methoxyphenyl]-2-[1-(3-phenyl-2-propynoyl)-4-piperidinyl]-1,3-thiazole-4-carboxamide;
2-(1-{[2-(allylsulfanyl)-3-pyridinyl]carbonyl}-4-piperidinyl)-*N*-[5-(aminocarbonyl)-2-methoxyphenyl]-1,3-thiazole-4-carboxamide;
*N*-[5-(aminocarbonyl)-2-methoxyphenyl]-2-{1-[(2-chlorophenyl)acetyl]-4-piperidinyl}-1,3-thiazole-4-carboxamide;
*N*-[5-(aminocarbonyl)-2-methoxyphenyl]-2-{1-[(3,4-dimethylphenoxy)acetyl]-4-piperidinyl}-1,3-thiazole-4-carboxamide;
*N*-[5-(aminocarbonyl)-2-methoxyphenyl]-2-[1-({[4-(dimethylamino)phenyl]amino}carbonothioyl)-4-piperidinyl]-1,3-thiazole-4-carboxamide;
   or
*N* [5-(aminocarbonyl)-2-methoxyphenyl]-2-(1-{[4-(2-pyridinyl)-1-piperazinyl]carbonyl}-4-piperidinyl)-1,3-thiazole-4-carboxamide.

More preferred compounds of the invention are:

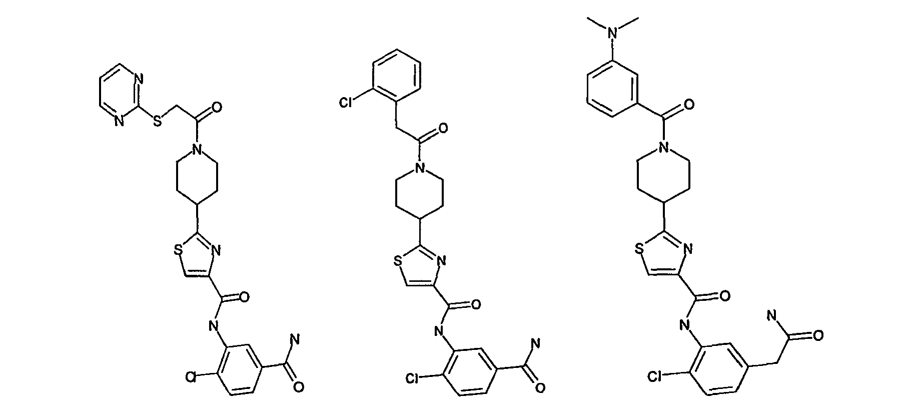

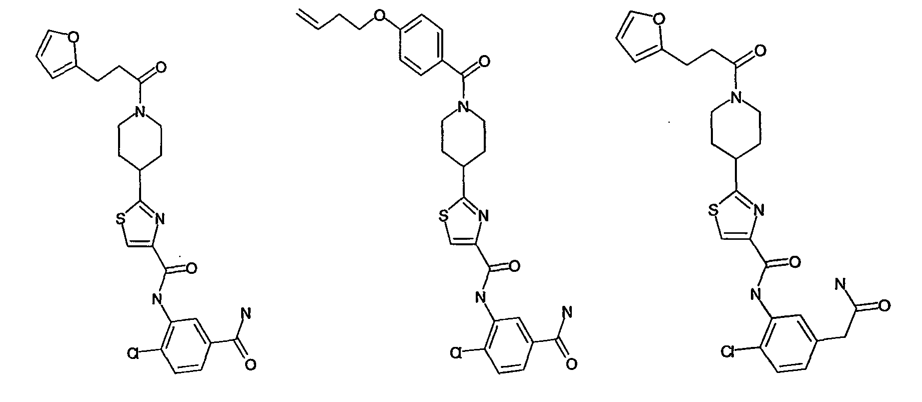

According to a further aspect of the present invention there is provided a compound of the present invention for use in a method of treatment of disease.

The compounds of the present invention may preferably be employed in the treatment of VEGF-mediated disorders such as endometriosis, various malignant and non-malignant tumours, psoriasis and other skin conditions, atheromatous disease, rheumatoid arthritis, macular degeneration and the complications of diabetes including retinopathy, nephropathy and neuropathy.

According to a further aspect of the present invention there is provided a compound of the present invention for use in therapy or diagnosis.

According to a further aspect of the present invention there is provided the use of a compound of the present invention for use in the manufacture of a medicament for treating a VEGF-mediated disorder, preferably endometriosis or malignant tumours.

According to a further aspect of the present invention there is provided a method of treating a disease mediated by VEGF, such as endometriosis, comprising administering to a patient in need of such treatment an effective dose of a compound of the present invention.

According to a further aspect of the present invention there is provided the use of a VEGF inhibitor for the manufacture of a medicament for treating acute macular degenerative disorder. Preferably, the VEGF inhibitor is a compound of the invention.

According to a further aspect of the present invention there is provided a method of treating acute macular degenerative disorder comprising administering to a patient in need of such treatment an effective dose of a VEGF inhibitor. Preferably, the VEGF inhibitor is a compound of the invention.

According to a further aspect of the present invention there is provided a pharmaceutical composition comprising a compound of the present invention in combination with a pharmaceutically acceptable excipient.

According to a further aspect of the present invention there is provided a VEGF inhibitor for topical administration for the treatment of acute macular degenerative disorder. Preferably, the VEGF inhibitor is a compound of the invention.

According to a further aspect of the present invention there is provided a topical system for the treatment of acute macular degenerative disorder comprising a VEGF inhibitor. Preferably, the VEGF inhibitor is a compound of the invention.

The agents described could be used alone or conjointly with treatments such as anti-hormone therapy, surgery, radiotherapy or chemotherapy.

Compounds of the present invention may be administered in a form suitable for oral use, for example a tablet, capsule, aqueous or oily solution, suspension or emulsion; for topical use including transmucosal and transdermal use, for example a cream, ointment, gel, aqueous or oil solution or suspension, salve, patch, plaster or as a component of a lubricant for a condom; for nasal use, for an example a snuff, nasal spray or nasal drops; for vaginal or rectal use, for example a suppository; for administration by inhalation, for example a finely divided powder or a liquid aerosol; for intra-ocular, sub-lingual or buccal use, for example a tablet or capsule; or for parenteral use (including intravenous, subcutaneous, intramuscular, intravascular or infusion), for example a sterile aqueous or oil solution or suspension, or incorporated in a biodegradable polymer. In general the above compositions may be prepared in a conventional manner using convention excipients, using standard techniques well known to those skilled in the art of pharmacy. The preferred modes of administration of the compound are oral or intravaginal. Oral administration is particularly preferred.

For oral administration, the compounds of the invention will generally be provided in the form of tablets or capsules or as an aqueous solution or suspension.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate, and lactose, while com starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredient is mixed with water or an oil such as peanut oil, liquid paraffin or olive oil.

For intramuscular, intraperitoneal, subcutaneous and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate. The compounds of the invention may also be provided in a biodegradable polymer, for example for use in conjunction with stents in surgery (e.g. adsorbed on a stent or applied directly to the site of the procedure for slow release of the active agent).

Topical administration is also a preferred administration. Topical administration, including transmucosal and transdermal use, includes administration by, for example, a cream, ointment, gel, aqueous or oil solution or suspension, salve, patch, plaster, tampon, sanitary napkin, or as a component of a lubricant for a condom. Topical systems include: compositions comprising a VEGF inhibitor in combination with a pharmaceutically acceptable excipient for topical administration; and administration devices including a composition comprising a VEGF inhibitor in combination with a pharmaceutically acceptable excipient for topical administration. Examples of compositions comprising a VEGF inhibitor in combination with a topically acceptable excipient are creams, ointments, gels, aqueous or oil solutions or suspensions, or salves. Examples of administration devices including a composition comprising a VEGF inhibitor in combination with a topically acceptable excipient are patches, plasters, tampons or sanitary napkins including the composition or condoms including a lubricant comprising the composition.

It will be appreciated that the dosage levels used may vary over quite a wide range depending upon the compound used, the severity of the symptoms exhibited by the patient and the patient's body weight. Without limitation to the present invention, typical dosages for treatment of endometriosis may be, for example, of the order of 1 microgram/kg/day to 1 milligram/kg/day, more preferably 10 microgram/kg/day to 0.25 milligram/kg/day orally. For intra-ocular administration, typical dosages would be of the order of 10 nanogram/kg/day to 1 microgram/kg/day. For treatment of tumours up to 5 milligrams/kg/day would be preferable.

For intra-vaginal administration typical dosages would be 10 micrograms/kg/day to 0.25 milligrams/kg/day.

Compounds of this invention may be prepared by the general reaction scheme, Reaction Scheme 1, wherein by "core" is meant the radical 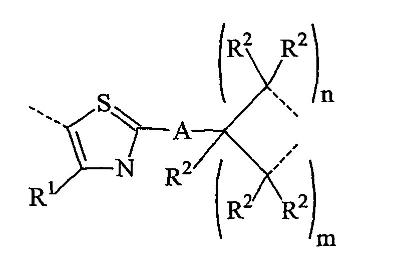 or

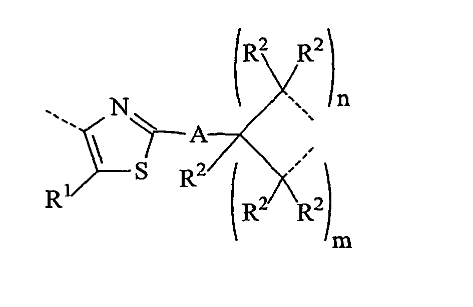

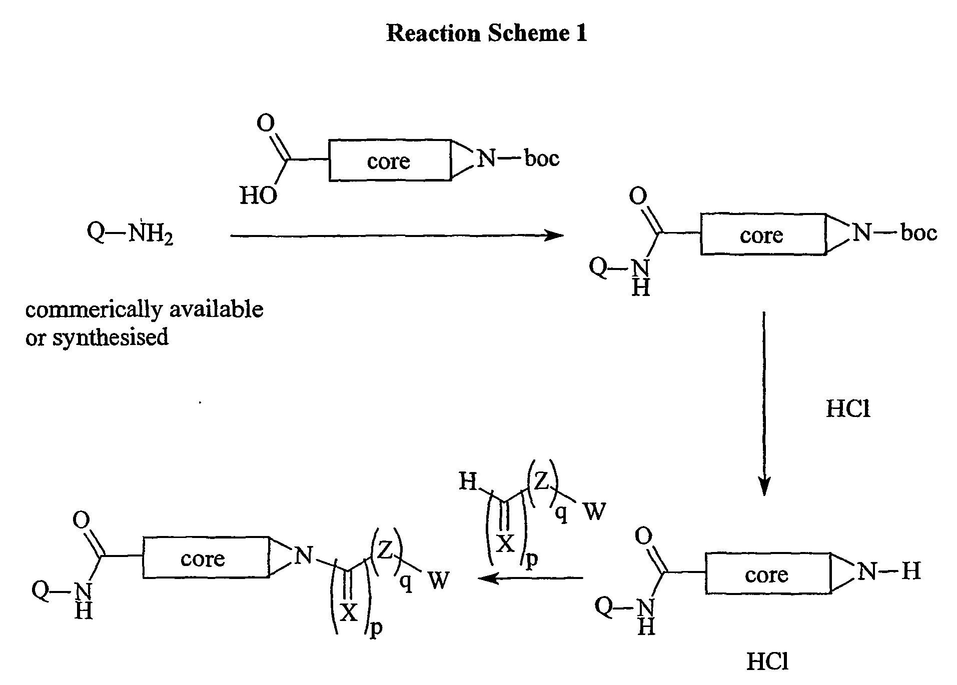

The invention is now further illustrated by means of the following Examples.

### Examples

### Synthesis of Specific Compounds of the Invention

### Examples 1-6

Preparation of *N*-[5-(aminocarbonyl)-2-methoxyphenyl]-2-{1-[(4,7-dimethylpyrazolo[5,1-*c*][1,2,4]triazin-3-yl)carbonyl]-4-piperidinyl}-1,3-thiazole-4-carboxamide; *N*-[5-(aminocarbonyl)-2-methoxyphenyl]-2-[1-(1-benzofuran-2-ylcarbonyl)-4-pipendinyl]-1,3-thiazole-4-carboxamide; *N-*[5-(aminocarbonyl)-2-methoxyphenyl]-2-[1-(3-phenyl-2-propynoyl)-4-piperidinyl] -1,3-thiazole-4-carboxamide; 2-(1-{[2-(allylsulfanyl)-3-pyridinyl]carbonyl}-4-piperidinyl)-*N-*[5-(aminocarbonyl)-2-methoxyphenyl]-1,3-thiazole-4-carboxamide; *N-*[5-(aminocarbonyl)-2-methoxyphenyl]-2-{1-[(2-chlorophenyl)acetyl]-4-piperidinyl}-1,3-thiazole-4-carboxamide; and *N*-[5-(aminocarbonyl)-2-methoxyphenyl]-2-{1-[(3,4-dimethylphenoxy)acetyl]-4-piperidinyl}-1,3-thiazole-4-carboxamide.

The above-mentioned compounds were synthesised by Reaction Scheme 2 below:

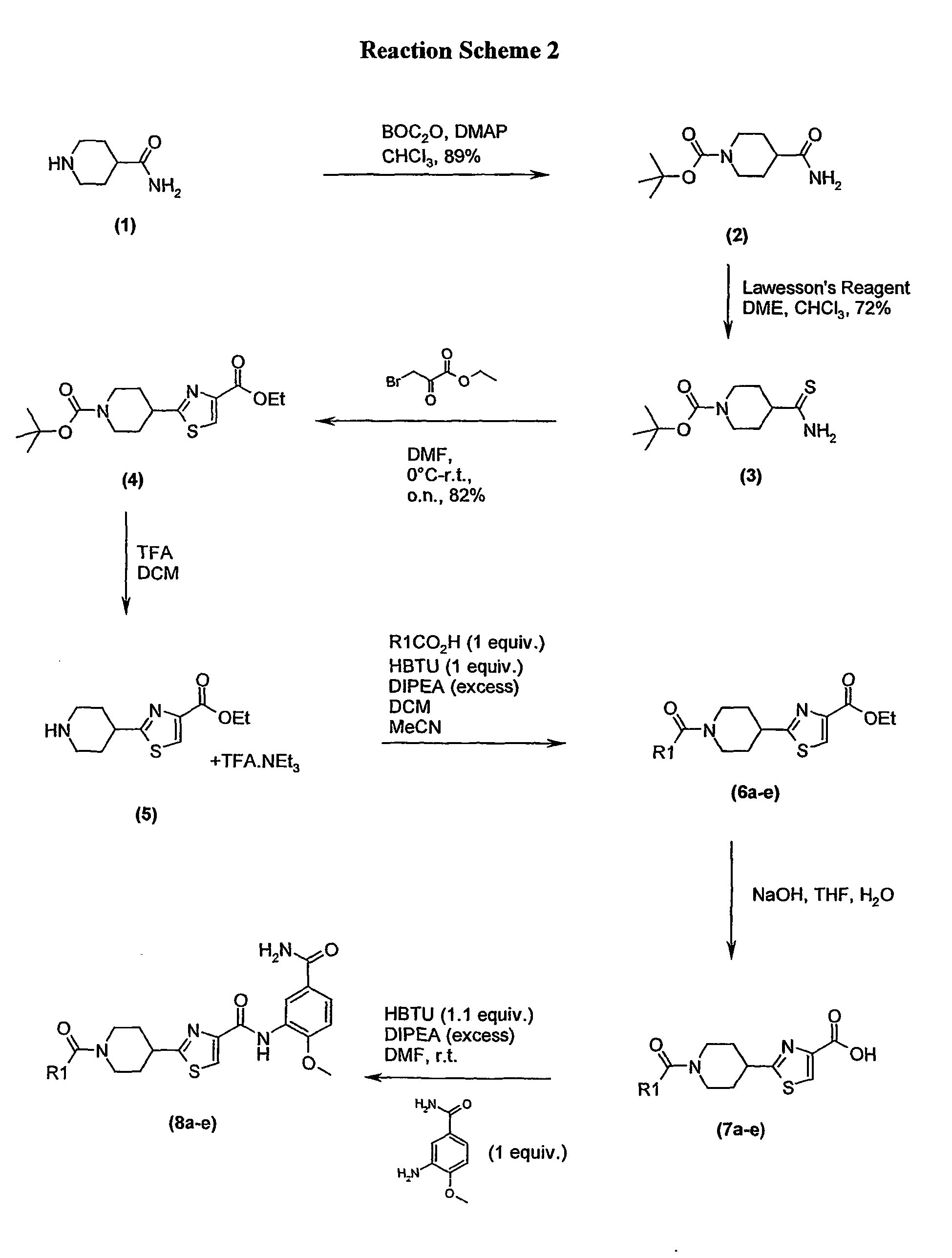

### 4-Carbamoyl-piperidine-1-carboxylic acid tert-butyl ester (2)

Isonipecotamide (1) (28.8 g, 0.22 mol) was suspended in chloroform (288 mL). To this was added 4-(dimethylamino)pyridine (DMAP) (23 mg, catalytic) followed by dropwise addition of a solution of BOC-anhydride (56 g, 0.26 mol, 1.14 equiv.) in chloroform (57 mL). The solution was stirred at room temperature for 1 h and then partitioned between chloroform and 10% citric acid solution. The organic phase was washed with citric acid solution and back extracted with chloroform. The combined organic extracts were washed with water, 10% brine and dried (MgSO₄). Filtration followed by evaporation of the filtrate gave the crude product as a pink solid. Crystallisation from ethyl acetate/hexane gave the title compound (2) as a colourless solid in 4 crops (45.5 g, 0.20 mol, 89%), m.p. 159-161°C (lit. 154-156°C).

### 4-Thiocarbamoyl-piperidine-1-carboxylic acid tert-butyl ester (3)

4-Carbamoyl-piperidine-1-carboxylic acid *tert-butyl* ester (2) (45.4 g, 0.199 mol), Lawesson's reagent (40.2 g, 0.099 mol, 0.5 equiv), 1,2-dimethoxyethane (DME) (500 mL) and chloroform (200 mL) were combined and stirred at room temperature. The course of the reaction was followed by tlc analysis (30% ethyl acetate/hexane) and on completion the reaction mixture was evaporated to dryness (glassy solid). The solid was dissolved in ethyl acetate and washed with half saturated potassium carbonate solution, dried (MgSO₄), filtered and concentrated to yield the title compound as a colourless solid. The crude product was crystallised from ethyl acetate and hexane to give the title compound (3) (35 g, 0.14 mol, 72%).

### 4-(4-Ethoxycarbonyl-thiazol-2-yl)-piperidine-carboxylic acid tert-butyl ester (4)

4-Thiocarbamoyl-piperidine-1-carboxylic acid *tert-*butyl ester (3) (25 g, 102 mmol) was dissolved in anhydrous *N,N-*dimethylformamide (DMF) (125 mL) and cooled to 0°C in an ice-bath. A solution of ethyl bromopyruvate (22.2 g, 14.3 mL, 114 mmol, 1.1 equiv) in anhydrous DMF (125 mL) was added dropwise with stirring. The reaction mixture was allowed to warm slowly to room temperature and stirred overnight. Triethylamine (25 mL) was added dropwise with stirring at the rate of 1 mL/g of thioamide used. The DMF was removed *in vacuo* keeping the temperature below 60°C. The resulting residue was partitioned between ethyl acetate (75 mL) and brine (100 mL). Sufficient water was added to ensure complete dissolution of the precipitated salts in the aqueous phase. The aqueous phase was extracted twice with ethyl acetate and the combined organic extracts washed successively with brine (x2), water (x2) and brine (x2). The organic phase was simultaneously dried with MgSO₄ and decolourised with finely divided charcoal. The mixture was filtered through Celite and concentrated *in vacuo* to give a yellow oil. Trituration with hexane yielded a yellow solid. This was diluted with an excess of hexane and cooled overnight to allow complete crystallisation of product. The product was collected by filtration, washed with hexane and dried *in vacuo* at room temperature. Recrystallisation from IPA/water gave the title compound (4) (28.33 g, 83 mmol, 82%).

### 2-Piperidine-4-yl-thiazole-4-carboxylic acid ethyl ester (5)

To a solution of 4-(4-ethoxycarbonyl-thiazol-2-yl)-piperidine-carboxylic acid *tert*-butyl ester (4) (5 g, 14.7 mmol) in dichloromethane (20 mL) at 0°C was added neat trifluoroacetic acid (TFA) (17 mL, 221 mmol, 15 equivalents) dropwise with stirring, under an inert atmosphere. On completion of addition the reaction mixture was allowed to warm to room temperature and stirring continued until deprotection complete (typically 3 hours, monitored by tlc, 1:1 hexane/ethyl acetate). On completion of reaction the mixture was concentrated *in vacuo* to remove TFA. Toluene (dioxan for (6f)) was then added and re-concentrated to further remove TFA - this was repeated 2-3 times to ensure maximum removal of TFA. The product was further dried *in vacuo* overnight to remove the last traces of TFA. The TFA salt of the free amine was dissolved in dichloromethane (10 mL), cooled to 0°C in an ice bath and treated with triethylamine (6.15 mL, 3 equiv). It was assumed a quantitative conversion of BOC-protected (4) to free amine (5).

### Compounds (6a-e)

To a solution of 2-piperidine-4-yl-thiazole-4-carboxylic acid ethyl ester (5) (14.7 mmol) in dichloromethane (10 mL) at 0°C was added acetonitrile (40 mL). To this solution was sequentially added the acid to be reacted (R1COOH - see Table 2) (14.7mmol, 1 equiv.), *N,N,N'N*'-tetramethyl-O-(*1H*-benzotriazole-1-yl) uronium hexafluorophosphate (HBTU) (14.7mmol, 5.57g, 1 equiv.), and *N,N-*diisopropylethylamine (DIPEA) (7.7 mL, 3 equiv.). The reaction mixture was stirred at room temperature for 48 h to allow for completion of reaction. After this time the reaction mixture was concentrated *in vacuo* to remove the solvent and the residue was suspended in dichloromethane (80 mL) and washed with brine (2 x 50 mL), water (50 mL), 10% citric acid (50 mL), brine, saturated sodium bicarbonate solution (50 mL) and finally brine. The organic layer was dried over MgSO₄ and treated with decolourising charcoal, filtered and concentrated *in vacuo.*

**Table 2**

| **id** | **R1CO₂H** | **yield** |
|---|---|---|
| **6a** | 2-chlorophenylacetic acid | not purified |
| **6b** | 3,4-dimethylphenoxyacetic acid | not purified |
| **6c** | 1-benzofuran-2-carboxylic acid | 65% (after chromatography) |
| **6d** | 3-phenylpropynic acid | not purified |
| **6e** | 2-(allylthio)nicotinic acid | not purified |
| **6f** | 4,7-dimethylpyrazolo[5,1-c][1,2,4]triazine-3-carboxylic acid | not purified |

### Compounds (7a-e)

Compound (6a-e) (13mmol) was dissolved in THF (35 mL) and water (23 mL) and cooled to 0°C. A solution of sodium hydroxide (1.04g, 26mmol, 2 equiv.) in water (20 mL) was added dropwise with stirring. The reaction was monitored by tlc analysis and when complete (typically 2 h) the reaction mixture was diluted with brine (30 mL) and washed with ether (100 mL). The reaction mixture was acidified using 20% citric acid solution. The acidic mixture was then extracted with a suitable organic solvent (dichloromethane or ethyl acetate) and when fully extracted the organic extracts were combined, dried over MgSO₄, filtered and concentrated *in vacuo* to yield essentially pure product.

**Table 3**

| **id** | **extraction solvent** | **yield** |
|---|---|---|
| **7a** | ethyl acetate | 58% (over two steps) |
| **7b** | ethyl acetate | 61% (over two steps) |
| **7c** | ethyl acetate | 80% |
| **7d** | dichloromethane | 41% (over two steps) |
| **7e** | ethyl acetate | 54% (over two steps) |
| **7f** | ethyl acetate | 53% (over two steps)* |

| | | |
|---|---|---|
| *product was isolated by repeated crystallisation from ethyl acetate | | |

### Compounds (8a-e)

Compound (7a-e) was dissolved in anhydrous DMF (0.58 M solution). 3-Amino-4-methoxybenzamide was dissolved in 10% DIPEA and anhydrous DMF (0.58 M solution). HBTU was dissolved in anhydrous DMF (0.64 M solution). The amine solution (0.3 ml, 0.175 mmol) was dispensed into an individual well in a 2.2 mL deep well plate using a Packard MPII robot. An Eppendorf multi-dispenser was used to dispense the acid solution (0.3 mL, 0.175 mmol, 1 equiv) and then to dispense the HBTU solution (0.3 mL, 0.19 mmol, 1.1 equiv). A further portion of DIPEA (0.05 mL) was added to the well, which was capped and shaken on an orbital shaker overnight. The reaction mixture was concentrated *in vacuo* (Genevac). The residue was dissolved in dichloromethane (1 mL) and given a sequence of aqueous washes using the MPII robot: 0.5 N HCl (0.7 mL), 10% potassium carbonate solution (0.7 mL) then water (0.7 mL). Finally the dichloromethane extract (0.7 mL) containing the product was concentrated and dried (Genevac) to constant mass.

**Table 4**

| **id** | **RRcode** | **DAD (254 nm)** | **ES+** |
|---|---|---|---|
| **8a** | **1506-03737** | 100% | 513 |
| **8b** | **1506-03914** | 97% | 523 |
| **8c** | **1506-01284** | 88% | 505 |
| **8d** | **1506-01461** | 90% | 489 |
| **8e** | **1506-02331** | 89% | 538 |
| **8f** | **1506-00581** | 86% | 535 |

### Example 7

### Preparation of N-[5-(aminocarbonyl)-2-methoxyphenyl]-2-[1-(1{[4-(dimethylamino)phenyl]amino}carbonothioyl)-4-piperidinyl]-1,3-thiazole-4-carboxamide;

*N*-[5-(aminocarbonyl)-2-methoxyphenyl]-2-[1-({[4-(dimethylamino)phenyl]amino carbonothioyl)-4-piperidinyl]-1,3-thiazole-4-carboxamide was prepared by the synthetic route set out in Reaction Scheme 3 below.

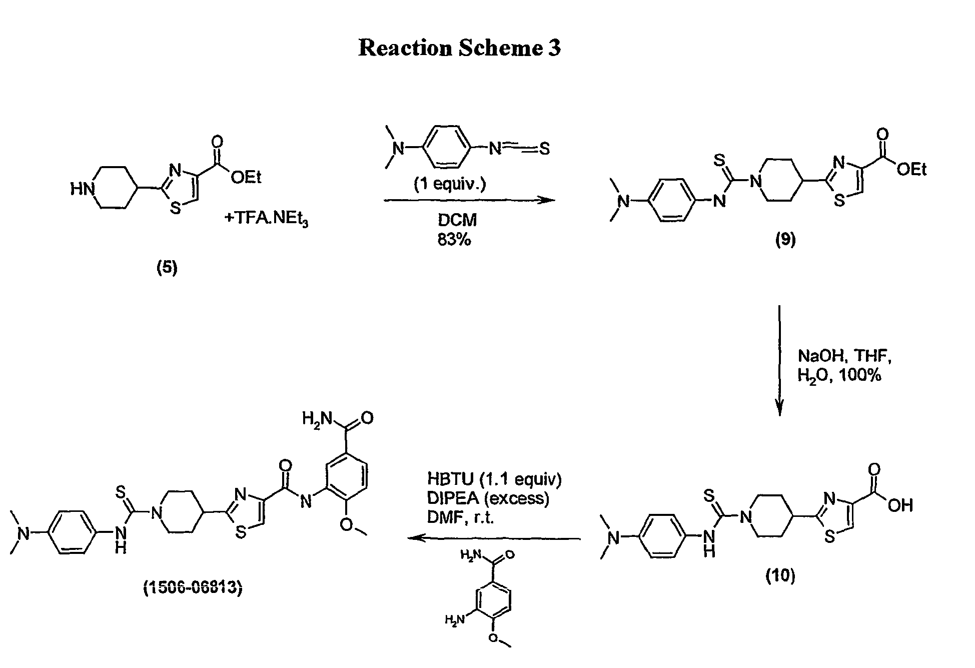

### 2-[1-(4-Dimethylamino-phenylthiocarbamoyl)-piperidine-4-yl]-thiazole-4-carboxylic acid ethyl ester (9)

To a solution of 2-piperidine-4-yl-thiazole-4-carboxylic acid ethyl ester (5) (14.7 mmol) in dichloromethane (10 mL) at 0°C was added dichloromethane (80 mL). To this was added 4-(dimethylamino)phenylisothiocyanate (14.7mmol, 1 equiv.). The reaction mixture was stirred at room temperature for 48 h to allow for completion of reaction. After this time the reaction mixture was diluted with dichloromethane (100 mL) and washed with water (2 x 100 mL) and brine (50 mL). The organic layer was dried over MgSO₄, filtered and concentrated *in vacuo* to yield the thiourea in 83% yield following chromatography.

### 2-[1-(4-Dimethylamino-phenylthiocarbamoyl)-piperidine-4-yl]-thiazole-4-carboxylic acid (10)

2-[1-(4-Dimethylamino-phenylthiocarbamoyl)-piperidine-4-yl]-thiazole-4-carboxylic acid ethyl ester (9) (13 mmol) was dissolved in THF (35 mL) and water (23 mL) and cooled to 0°C. A solution of sodium hydroxide (1.04g, 26 mmol, 2 equiv.) in water (20 mL) was added dropwise with stirring. The mixture was stirred for 2 h at r.t. The mixture was diluted with brine (30 mL) and washed with ether (100 mL). The reaction mixture was acidified using 20% citric acid solution. The acidic mixture was extracted with ethyl acetate and when fully extracted the organic extracts were combined, dried over MgSO₄, filtered and concentrated *in vacuo* to yield the title compound (10) in quantitative yield.

### N-[5-(aminocarbonyl)-2-methozyphenyl]-2-[1-({[4-(dimethylamino)phenyl]amino}carbonothioyl)-4-piperidinyl]-1,3-thiazole-4-carboxamide

2-[1-(4-Dimethylamino-phenylthiocarbamoyl)-piperidine-4-yl]-thiazole-4-carboxylic acid (10) was dissolved in anhydrous DMF (0.58 M solution). 3-Amino-4-methoxybenzamide was dissolved in 10% DIPEA and anhydrous DMF (0.58 M solution). HBTU was dissolved in anhydrous DMF (0.64 M solution). The amine solution (0.3 ml, 0.175 mmol) was dispensed into an individual well in a 2.2 mL deep well plate using a Packard MPII robot. An Eppendorf multi-dispenser was used to dispense the acid solution (0.3 mL, 0.175 mmol, 1 equiv) and then to dispense the HBTU solution (0.3 mL, 0.19 mmol, 1.1 equiv). A further portion of DIPEA (0.05 mL) was added to the well, which was capped and shaken on an orbital shaker overnight. The reaction mixture was concentrated *in vacuo* (Genevac). The residue was dissolved in dichloromethane (1 mL) and given a sequence of aqueous washes using the MPII robot: 0.5 N HCl (0.7 mL), 10% potassium carbonate solution (0.7 mL) then water (0.7 mL). Finally the dichloromethane extract (0.7 mL) containing the product was concentrated and dried (Genevac) to constant mass to give *N*-[5-(aminocarbonyl)-2-methoxyphenyl]-2-[1-({[4-(dimethylaxnino)phenyl] amino}carbonothioyl)-4-piperidinyl]-1,3-thiazole-4-carboxamide (DAD 75% (254nm), ES+ 539).

### Example 8

### Preparation of N [5-(aminocarbonyl)-2-methoxyphenyl]-2-(1-{[4-(2-pyridinyl)-1-piperazinyl]carbonyl}-4-piperidinyl)-1,3-thiazole-4-carboxamide

*N-*[5-(aminocarbonyl)-2-methoxyphenyl]-2-(1-{[4-(2-pyridinyl)-1-piperazinyl]carbonyl}-4-piperidinyl)-1,3-thiazole-4-carboxamide was prepared by the synthetic route set out in Reaction Scheme 4 below.

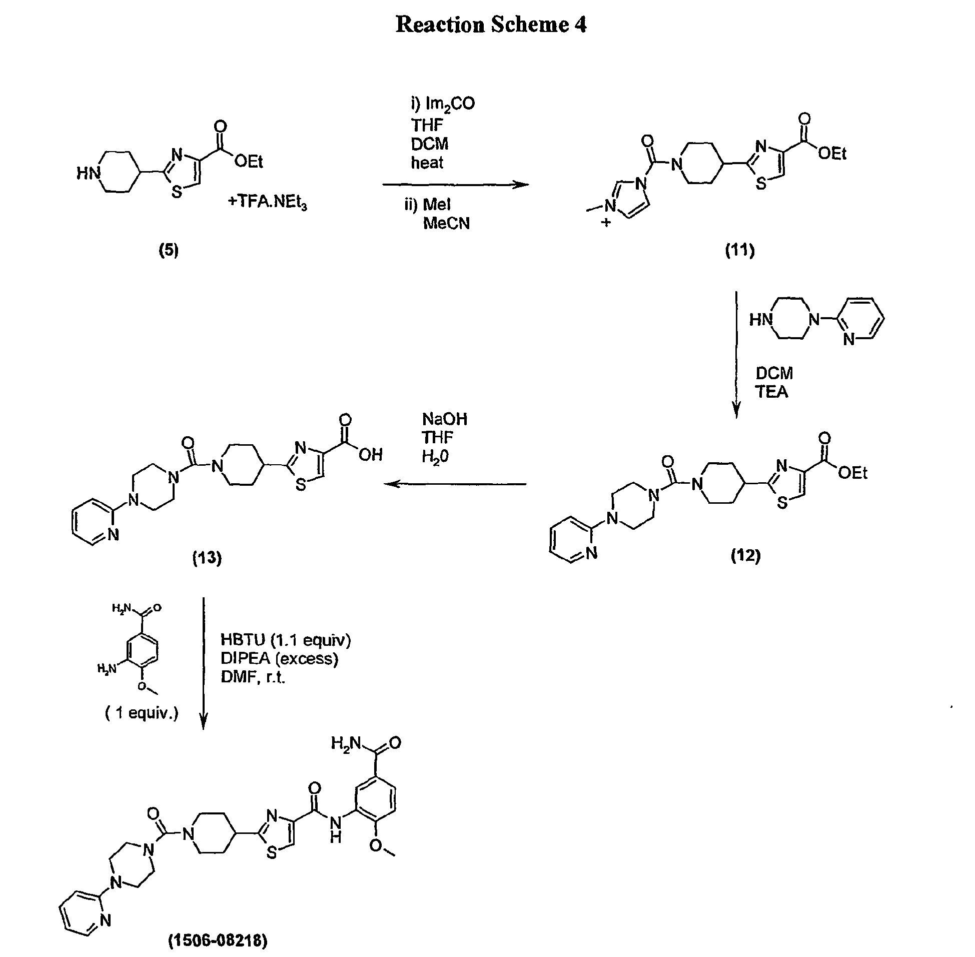

### 3-[4-(4-Ethoxycarbonyl-thiazol-2-yl)-piperidine-1-carbonyl]-1-methyl-3H-imidazol-1-ium (11)

A solution of 2-piperdine-4-yl-thiazole-4-carboxylic acid ethyl ester (5) (14.7 mmol) in dichloromethane (15 mL) was added dropwise to a suspension of carbonyldiimidazole in tetrahydrofuran (15 mL). The mixture was heated at reflux overnight then cooled to room temperature. The solvent was removed in vacuo and the residue was dissolved in dichloromethane (80 mL), washed with water and dried over MgSO₄ and concentrated *in vacuo.* The residue was dissolved in acetontrile and methyl iodide added (59 mmol). The mixture was stirred overnight and concentrated to give the title compound which was used without purification.

### 2-[1-(4-Pyridine-2-yl-piperazine-1-carbonyl)-piperidin-4-yl]-thiazole-4-carboxylic acid ethyl ester (12)

3-[4-(4-Ethoxycarbonyl-thiazol-2-yl)-piperidine-1-carbonyl]-1-methyl-3*H*-imidazol-1-ium (11) was taken up in dichloromethane (75 mL) and 1-(2-pyridyl)piperazine (14.7 mmol, 1 equiv.) and triethylamine (14.7 mmol, 1 equiv.) added. The mixture was stirred overnight and diluted with dichloromethane. The mixture was washed with water and brine, dried and concentrated *in vacuo* to yield the title compound which was used without purification.

### 2-[1-(4-Pyridine-2-yl-piperazine-1-carbonyl)-piperidin-4-yl]-thiazole-4-carbozylic acid ethyl ester (13)

The ethyl ester (12) was taken up in tetrahydrofuran (40 mL) / water (20 mL) and sodium hydroxide (29.4 mmol) in water (20 mL) added. The mixture was stirred for 2 h at room temperature. The mixture was then extracted with ether and the aqueous phase acidified with 10% citric acid solution. The aqueous phase was extracted with ethyl acetate and the combined extracts washed with brine, dried and concentrated *in vacuo.* Precipitated product remaining in the aqueous layer was filtered, dried and added to the residue. The title compound (13) was obtained as a colourless solid [total yield 59% (3 steps)].

### N-[5-(aminocarbonyl)-2-methoxyphenyl]-2-(1-{[4-(2-pyridinyl)-1-piperazinyl]carbonyl}-4-piperidinyl)-1,3-thiazole-4-carboxamide

2-[1-(4-Pyridine-2-yl-piperazine-1-carbonyl)-piperidin-4-yl]-thiazole-4-carboxylic acid ethyl ester (13) was dissolved in anhydrous DMF (0.58 M solution). 3-Amino-4-methoxybenzamide was dissolved in 10% DIPEA and anhydrous DMF (0.58 M solution). HBTU was dissolved in anhydrous DMF (0.64 M solution). The amine solution (0.3 ml, 0.175 mmol) was dispensed into an individual well in a 2.2 mL deep well plate using a Packard MPII robot. An Eppendorf multi-dispenser was used to dispense the acid solution (0.3 mL, 0.175 mmol, 1 equiv) and then to dispense the HBTU solution (0.3 mL, 0.19 mmol, 1.1 equiv). A further portion of DIPEA (0.05 mL) was added to the well, which was capped and shaken on an orbital shaker overnight. The reaction mixture was concentrated *in vacuo* (Genevac). The residue was dissolved in DCM (1 mL) and given a sequence of aqueous washes using the MPII robot: 0.5 N HCl (0.7 mL), 10% potassium carbonate solution (0.7 mL) then water (0.7 mL). Finally the DCM extract (0.7 mL) containing the product was concentrated and dried (Genevac) to constant mass to give N-[5-(aminocarbonyl)-2-methoxyphenyl]-2-(1-{ [4-(2-pyridinyl)-1-piperazinyl]carbonyl}-4-piperidinyl)-1,3-thiazole-4-carboxamide (DAD 100% (254nm), ES+ 550).

### Activity Assays

Activities of compounds of the invention were tested using the following assays. The results of the assays are set out in tables 5 and 6 below. For some compounds the assays were repeated to give the multiple results shown in the table.

Initially the compounds were assayed for inhibition of VEGF in a medium throughput ELISA assay before detailed assessment in the HUVEC and VEGF binding assays in examples in 9 and 10. The data from this initial assessment is presented in table 5 in the column labelled "Inhibition %".

### Example 9 - Human umbilical vein endothelial cells (HUVEC) proliferation assay

The HUVEC assay measures the potency of test substances to inhibit *in vitro* proliferation of human umbilical vein endothelial cells (HUVEC) when co-stimulated with recombinant human vascular endothelial growth factor (rhVEGF).

HUVEC are grown under defined conditions (EGM-2 medium, 37°C, 5 % CO₂ and 95 % humidity). They are seeded into 48-well plates using EBM medium with only 1 % serum and incubated for 24 h. This is to ensure that cells are not stimulated before treatment with VEGF and test compound.

Test compounds are diluted in medium to concentrations between 0.05 and 50 µM and dosed together with VEGF₁₆₅ at 12 ng/ml. This VEGF concentration was determined in a VEGF-dose-response curve to be just sub-optimal for maximum cell proliferation. Cells are incubated for 48 h at above conditions and viable cell density is measured using a tetrazolium compound (MTS). Viable cells reduce MTS into a soluble formazan product, which has an absorbance maximum at 490 nm. The absorbance is directly proportional to cell density.

| | |
|---|---|
| Control values are: | EBM + 1 % serum (no VEGF) ⇒ Minimum or Blank |
| | EBM + 1 % serum (12 ng/ml) ⇒ Maximum |

### Example 10 - VEGF-binding ELISA

The potency of test substances as a VEGF-neutralising moiety is measured in an ELISA format. This assay measures the solution-phase interaction between rh VEGF₁₆₅-biotin and test sample. Unbound VEGF₁₆₅-biotin is then immobilised on a solid-phase anti-hVEGF antibody (R&D systems MAB293). The biotin signal is detected with streptavidin-alkaline phosphatase, which gives a colorimetric signal when incubated with p-nitrophenyl phosphate. Plates are read in a spectrophotometer at 405 nm.

Any test compound that binds to VEGF165-biotin and prevents it from binding to the antibody will lower the color signal and be recognized as a "hit". Hits were defined as compounds that show> 60 % inhibition.

| | |
|---|---|
| Control values are: | VEGF165-biotin + assay buffer => 0 % inhibition |
| | Soluble VEGF receptor (sflt @ 4 nM) ==> 70 % inhibition |

The primary screening of all library compounds was done at a compound concentration of 50 µM, followed by measuring dose-response effects of "hits" between 0.05 and 500 µM for the calculation of IC₅₀ values.

The compounds were dissolved in DMSO and it was shown that the solvent has no effect on the assay at this concentration.

### Example 11- Additional Assays

Compounds 1633-00382 and 1633-02177 were tested for selectivity by determining their effect on the following assays:
- Basic Fibroblast Growth Factor (FGF)-Induced cell proliferation (Gospodarowicz D, Brown KD, Birdwell CR & Zetter BR (1978) J. Cell. Biol. 77: 774-788);
- Epidermal Growth Factor (EGF) radioligand binding assay (Dittadi R, Gion M, Brazzale A., Bruscagnin G. (1990) Clin. Chem. 36: 849-854; Massague J (1983) J. Biol. Chem. 258: 13614-13620); and
- Platelet-Derived Growth Factor (PDGF) radioligand binding assay (Williams LT, Tremble PM, Lavin MF & Sunday ME (1984) J. Biol. Chem. 259 5287-5294

Compounds 1633-00382 and 1633-02177 did not inhibit EGF and PDGF binding to human A431 and mouse 3T3 cells respectively.

In addition, compound 1633-02177 demonstrated significant anti-proliferative activity against basic FGF with an estimated value of 0.406µM. This activity was accompanied by a moderate but not significant cytotoxicity at 10µM.

All documents cited herein are incorporated by reference in their entirety.

**Table 5**

| Activities of compounds of the invention | | | | | |
|---|---|---|---|---|---|
| **Compound no.** | **Metris ID no.** | **Inhibition (%)** | **IC50 VEGF (µM)** | **IC50 HUVEC (µM)** | **Structure** |
| 1506-00581 | M2025-0001 | 88 | 4.4 | 30 | 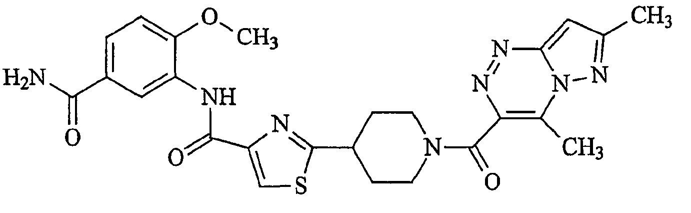 |
| | | | 5.0 | 31 | |
| 1506-01284 | M2025-0002 | 85 | 11.0 | 10.1 | 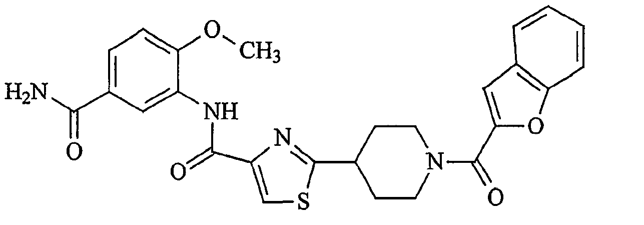 |
| | | | 13.5 | 12.6 | |
| 1506-01461 | M2025-0003 | 88 | 4 | 11 | 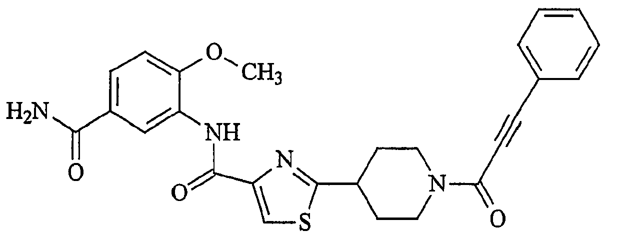 |
| | | 75 | | | |
| | | 83 | | | |
| 1506-02331 | M2025-0004 | 43 | 6.5 | 22.8 | 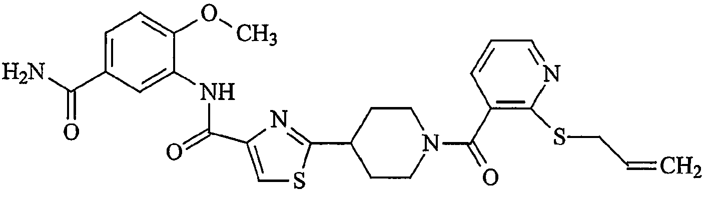 |
| | | 73 | | | |
| | | 79 | | | |
| 1506-03737 | M2025-0005 | 60 | 214 | 9 | 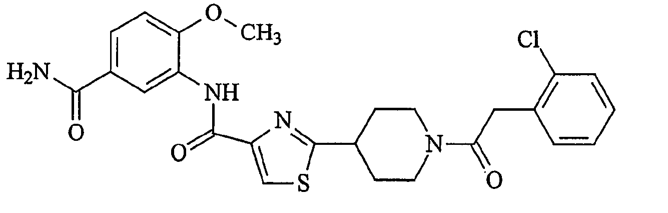 |
| | | 70 | 8 | | |
| | | 74 | | | |
| 1506-03914 | M2025-0006 | 77 | 4.5 | 17 | 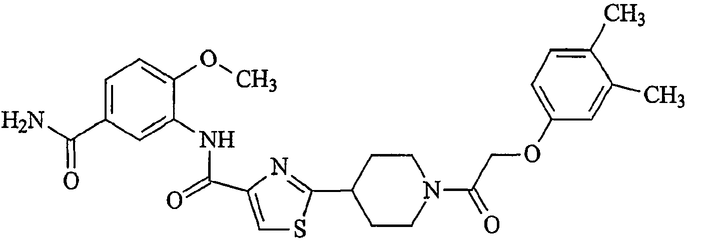 |
| | | 70 | | | |
| | | 81 | | | |
| 1506-06813 | M2025-0007 | 88 | 7.3 | 9.7 | 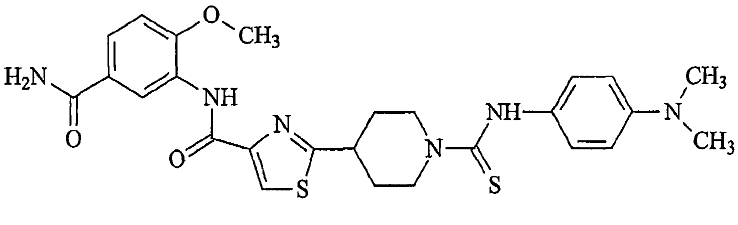 |
| | | | 1.5 | 8.2 | |
| 1506-08218 | M2025-0008 | 52 | 14.8 | 15.4 | 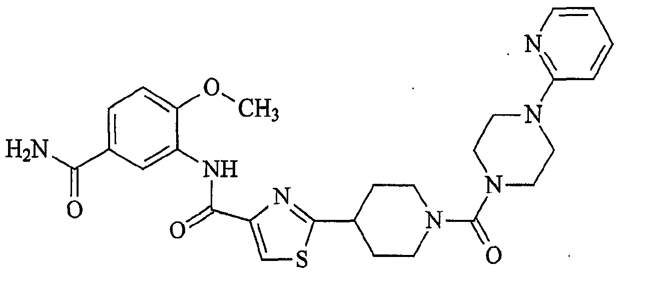 |
| | | 60 | | | |
| | | 67 | | | |
| 1506-00404 | | 72 | 153 | 10.4 | 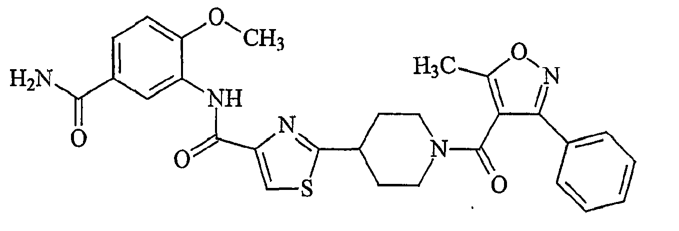 |
| | | -49 | | | |
| | | -35 | | | |
| 1506-00935 | | 65 | 29 | | 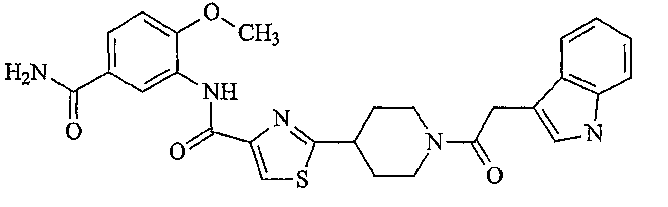 |
| | | 51 | | | |
| | | 31 | | | |
| 1506-01112 | | 11 | 16.7 | 26 | 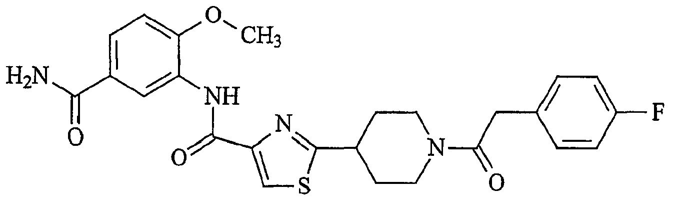 |
| | | 17 | | | |
| | | 55 | | | |
| 1506-01638 | | 89 | 71 | 5.4 | 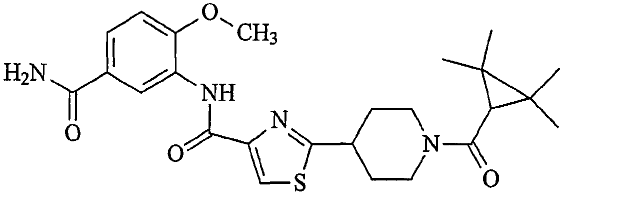 |
| | | | >100 | 6.0 | |
| 1506-01810 | | -43 | 136 | 5.6 | 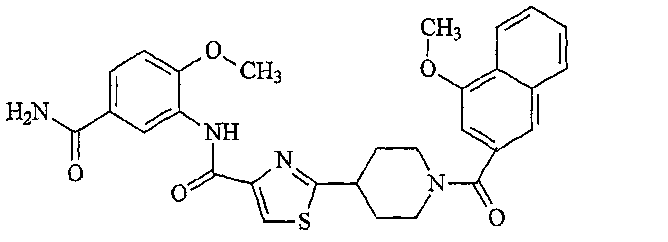 |
| | | -10 | | | |
| | | -14 | | | |
| 1506-02159 | | 46 | 128 | | 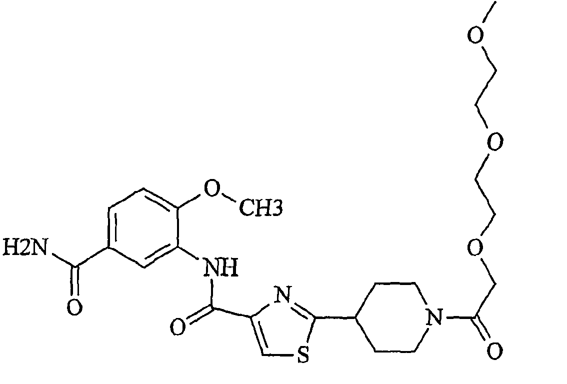 |
| | | -20 | | | |
| | | -83 | | | |
| 1506-03039 | | 63 | 105 | 4.2 | 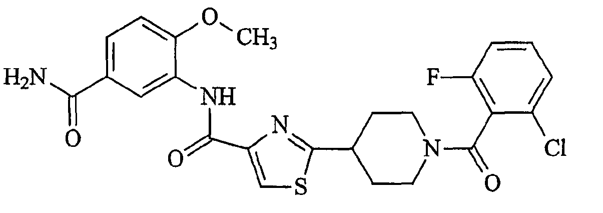 |
| | | -13 | | | |
| | | -67 | | | |
| 1506-03211 | | 64 | 132 | | 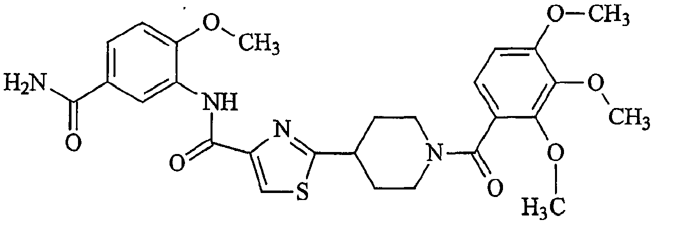 |
| | | -24 | | | |
| | | -80 | | | |
| 1506-03388 | | 93 | 6.6 | 23 | 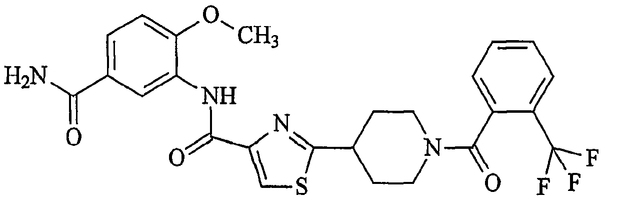 |
| | | 87 | | | |
| | | 90 | | | |
| 1506-03560 | | 42 | 3.6 | 16.4 | 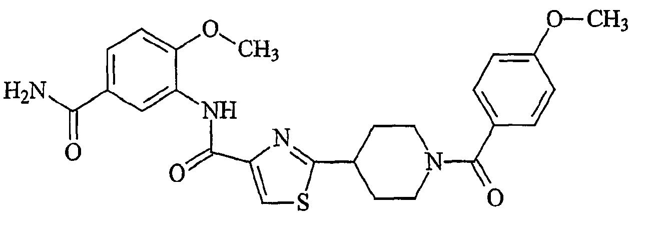 |
| | | 88 | | | |
| | | 85 | | | |
| 1506-04091 | | 71 | 16.0 | | 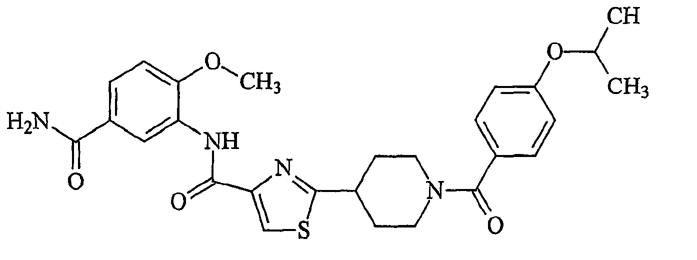 |
| | | 56 | 15.5 | | |
| | | 19 | | | |
| 1506-07095 | | 61 | 173 | | 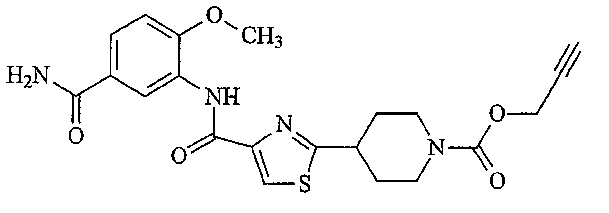 |
| | | -37 | | | |
| | | -63 | | | |
| 1506-07554 | | 84 | 142 | 35 | 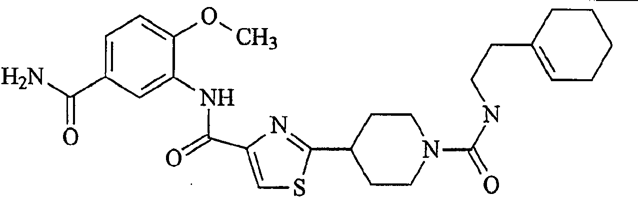 |
| | | -15 | | | |
| | | -83 | | | |
| 1506-08674 | | 64 | 11.0 | | 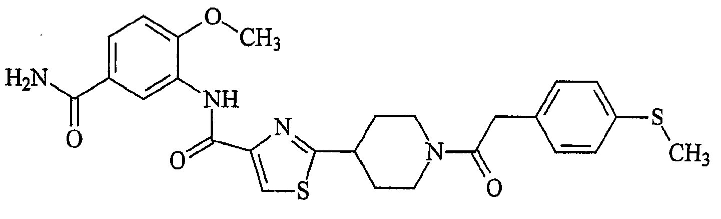 |
| | | 84 | | | |
| | | 36 | | | |
| 1506-08772 | | 91 | 54 | 25 | 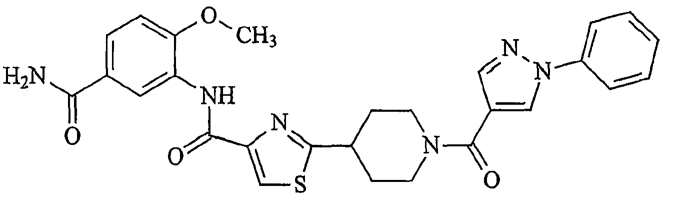 |
| | | | 126 | | |

**Table 6**

| Activites of compounds of the invention | | | |
|---|---|---|---|
| **Compound no.** | **IC50 VEGF (µM)** | **IC50 HUVEC (µM)** | **Structure** |
| 1633-00382 | 0.655 | 0.938 | 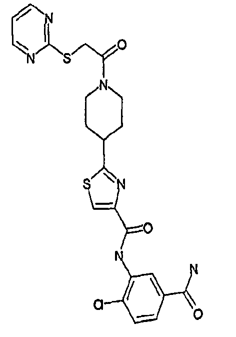 |
| | 3.485* | 16* | |
| | 4** | 20** | |
| | 1*** | 12*** | |
| 1633-00375 | 5 | 0.448 | 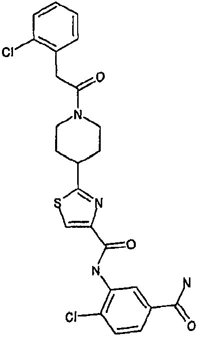 |
| | 9**$ | 17**$ | |
| 1633-02177 | 12 | 0.712 | 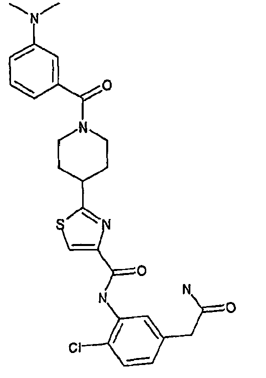 |
| | 18** | 0.478** | |
| 1633-00377 | 5 | 0.222 | 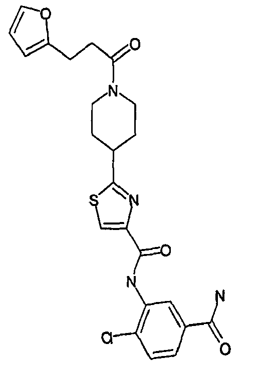 |
| 1633-00884 | 30 | 0.649 | 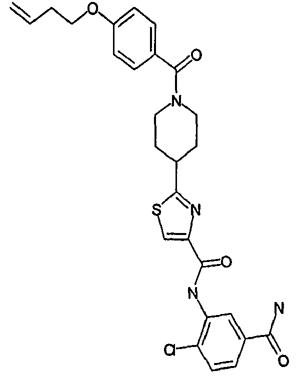 |
| 1633-01861 | 13 | 0.803 | 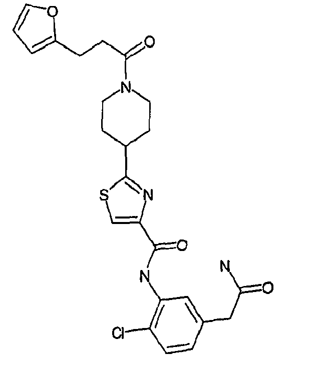 |

| | | | |
|---|---|---|---|
| * re-test 3 months later ** tested after re-synthesised *** re-tested in full-concentration response assays $ solubility problems | | | |

## Claims

1. A compound of formula (I) or formula (II): 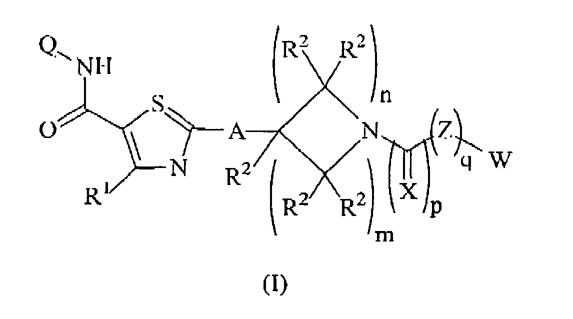 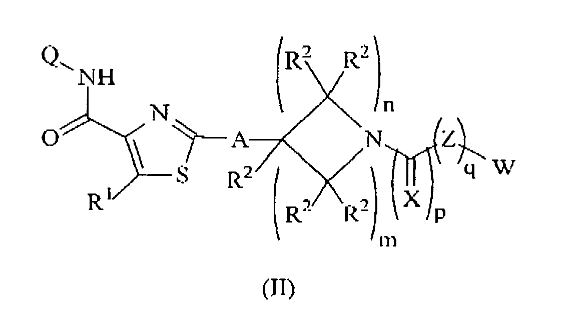 wherein:
Q is an optionally substituted C₁₋₁₀alkyl, C₁₋₁₀alkenyl, C₃₋₁₂cycloalkyl, C₆₋₁₂aryl, C₆₋₁₂arylC₁₋₁₀alkyl, C₁₋₁₀alkoxy, C₆₋₁₂aryloxy, C₆₋₁₂arylC₁₋₁₀alkyloxy, C₁₋₁₀alkylthio, C₆₋₁₂arylC₁₋₁₀alkylihio, amino, C₁₋₁₀amino, diC₁₋₁₀alkylamino, carboxyl, carboxylC₁₋₁₀alkyl, esterified carboxyl, C₁₋₁₀alkylsulfoxyl, C₁₋₁₀alkylsulfonyl, nitro, carbonitrile, carbo-C₁₋₁₀alkoxy, carbo-C₆₋₁₂aryloxy, or a heterocyclic group containing 5 to 12 atoms and 1, 2, 3 or 4 heteroatoms selected from N, O and S;
A is a single bond, cyclic C₃₋₁₂alkylene or acyclic C₁₋₁₆alkylene;
X is O or S;
Z is O, S or NR³;
p is 0 or 1;
q is 0 or 1;
n is an integer from 0 to 10;
m is an integer from 0 to 10;
W is an optionally substituted C₁₋₁₀alkyl, (C₁₋₁₀alkenyl, C₃₋₁₂cycloalkyl, C₆-₁₂aryl, C₆₋₁₂arylC₁₋₁₀alkyl, C₁₋₁₀alkoxy, C₆₋₁₂aryloxy, C₆₋₁₂arylC₁₋₁₀alkyloxy, C₁₋₁₀alkylthio, C₆₋₁₂arylC₁₋₁₀alkylthio, amino, C₁₋₁₀amino, diC₁₋₁₀alkylamino, carboxyl, carboxylC₁₋₁₀alkyl, esterified carboxyl, C₁₋₁₀alkylsulfoxyl, C₁₋₁₀alkylsulfonyl, nitro, carbonitrile, carbo-C₁₋₁₀alkoxy, carbo-C₆₋₁₂aryloxy, or a heterocyclic group containing 5 to 12 atoms and 1, 2, 3 or 4 heteroatoms selected from N, O and S;
R¹ is H or C₁₋₁₀alkyl;
R² is independently II or C₁₋₁₀alkyl; and
R³ is II or C₁₋₁₀alkyl;
or a pharmaceutically acceptable derivative thereof,
wherein, when a group is substituted, it may be substituted with a halogen atom or a halomethyl group such as CF₃ or CCl₃; an oxygen containing group such as oxo, hydroxy, carboxy, carboxyalkyl, C₁₋₁₀alkoxy, C₁₋₁₀alkoyl, C₁₋₁₀alkoyloxy, C₆₋₁₂aryloxy, C₆₋₁₂aryloyl or C₆₋₁₂aryloyloxy; a nitrogen containing group such as amino, amido, alkylamino, dialkylamino, cyano, azide, nitrato, nitro, -C(O)-NH₂, -C(O)NHR⁴ or -C(O)NR⁴₂; a sulphur containing group such as thiol, C₁₋₁₀alkylthiol, sulphonyl, sulphoxide, -S(O)₂H, -S(O)₂R⁴, -S(O)-H or -S(O)-R⁴; a heterocyclic group containing 5 to 12 atoms and 1, 2, 3 or 4 heteroatoms selected from N, O and S, such as thienyl, furanyl, pyrrolyl, imidazolyl, pyrazolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolyl, isoquinolyl, naphihridinyl, cinnolinyl, quinazolinyl, pyridopyridyl, benzoxazinyl, quinoxadinyl, chromenyl, chromanyl, isochromanyl, carbolinyl, oxadiazolyl, thiadiazolyl, triazolyl, tetrazolyl or pyrimidinyl; a C₁₋₁₀alkyl group, which may itself be substituted; or an C₆₋₁₂aryl group, which may itself be substituted, such as phenyl or substituted phenyl,
where R⁴ is independently optionally substituted C₁₋₁₀alkyl or C₆₋₁₂aryl,

2. The compound of claim 1 of formula (II).

3. The compound of claim 1 or claim 2 wherein A is a single bond.

4. The compound of any of claims 1 to 3 wherein X is O.

5. The compound of any of claims 1 to 3 wherein X is S and Z is NR³.

6. The compound of any of claims 1 to 5 wherein R³ is 11.

7. The compound of any of claims 1 to 6 wherein p = 1.

8. The compound of any of claims 1 to 7 wherein q = 0.

9. The compound of any of claims 1 to 8 wherein the sum n + m is an integer from 2 to 10.

10. The compound of any of claims 1 to 9 wherein the sum n + m is 4.

11. The compound of any of claims I to 10 wherein n = 2 and m - 2.

12. The compound of any of claims 1 to 11 wherein R¹ is H.

13. The compound of any of claims 1 to 12 wherein R² is H.

14. The compound of any of claims I to 13 wherein Q is an optionally substituted C₁₋₁₀alkyl, C₁₋₁₀alkenyl, C₃₋₁₂cycloalkyl, C₆₋₁₂aryl, C₆₋₁₂arylC₁₋₁₀alkyl, carboxyl, carboxylC₁₋₁₀alkyl, esterified carboxyl, C₁₋₁₀alkylsulfonyl or a heterocyclic group containing 5 to 12 atoms and 1, 2, 3 or 4 heteroatoms selected from N, O and S.

15. The compound of any of claims 1 to 14 wherein Q comprises an optionally substituted C₆₋₁₂aryl or a heterocyclic group containing 5 to 12 atoms and 1, 2, 3 or 4 heteroatoms selected from N, O and S.

16. The compound of any of claims 1 to 15 wherein Q is an optionally substituted C₆₋₁₂aryl or a heterocyclic group containing 5 to 12 atoms and 1, 2, 3 or 4 heteroatoms selected from N, O and S.

17. The compound of any of claims 1 to 15 wherein Q comprises an optionally substituted phenyl.

18. The compound of any of claims 1 to 17 wherein Q is an optionally substituted phenyl.

19. The compound of any of claims 1 to 16 wherein Q is an optionally substituted Furanyl.

20. The compound of any of claims 1 to 16 wherein Q is an optionally substituted thienyl.

21. The compound of any of claims 1 to 16 wherein Q is a radical selected from the radicals set out in table 1.
**Table 1**
| **Q** | **Radical** |
|---|---|
| Q¹ | 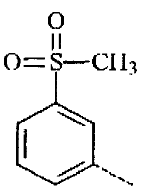 |
| Q² | 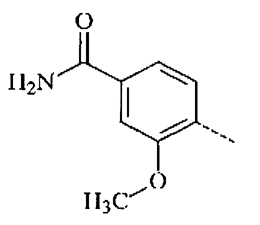 |
| Q³ | 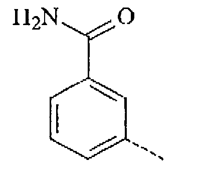 |
| Q⁴ | 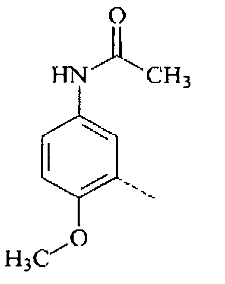 |
| Q⁵ | 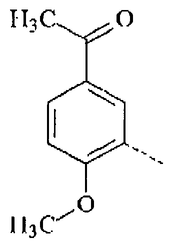 |
| Q⁶ | 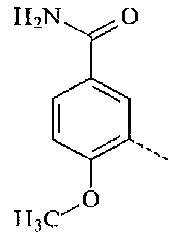 |
| Q⁷ | 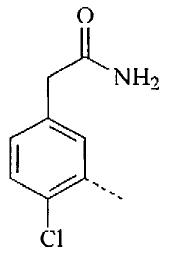 |
| Q⁸ | 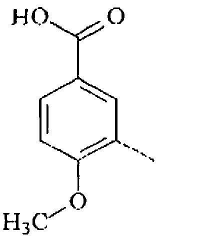 |
| Q⁹ | 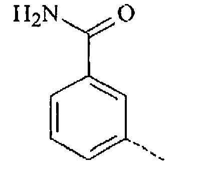 |
| Q¹⁰ | 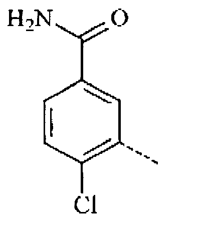 |
| Q¹¹ | 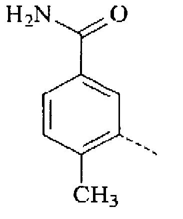 |
| Q¹² | 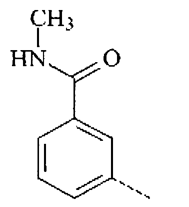 |
| Q¹³ | 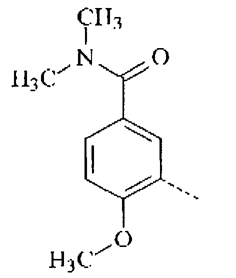 |
| Q¹⁴ | 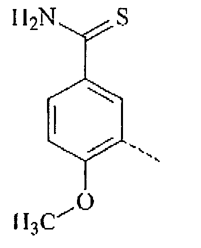 |
| Q¹⁵ | 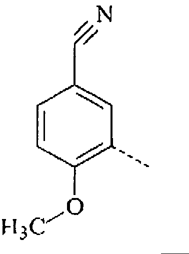 |
| Q¹⁶ | 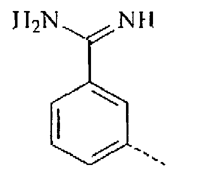 |
| Q¹⁷ | 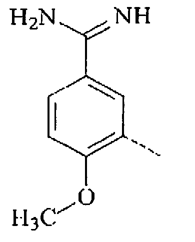 |
| Q¹⁸ | 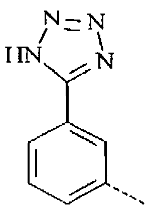 |
| Q¹⁹ | 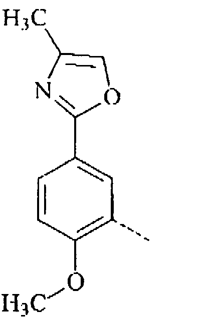 |
| Q²⁰ | 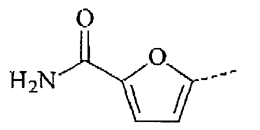 |
| Q²¹ | 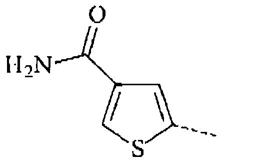 |
| Q²² | 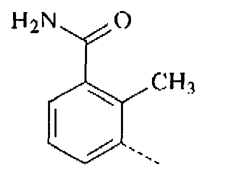 |
| Q²³ | 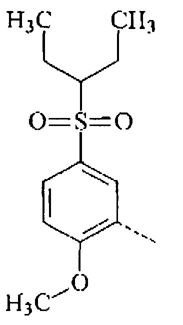 |
| Q²⁴ | 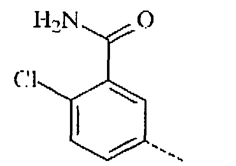 |
| Q²⁵ | 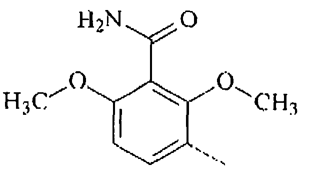 |
| Q²⁶ | 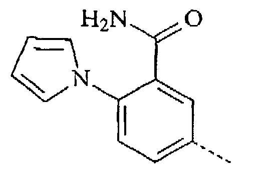 |

22. The compound of claim 21 wherein Q is Q₆, Q₇ or Q₁₀.

23. The compound of any of claims 1 to 22 wherein W is an optionally substituted C₁₋₁₀alkyl, C₁₋₁₀alkenyl, C₃₋₁₂cycloalkyl, C₆₋₁₂aryl, C₆₋₁₂arylC₁₋₁₀alkyl, C₁₋₁₀alkoxy, C₆₋₁₂aryloxy, C₆₋₁₂arylC₁₋₁₀alkyloxy, C₁₋₁₀alkylthio, C₆₋₁₂arylC₁₋₁₀alkylthio, carboxyl, carboxylC₁₋₁₀alkyl, esterified carboxyl, C₁₋₁₀alkylsulfonyl, carbo-C₁₋₁₀alkoxy, carbo-C₆₋₁₂aryloxy or a heterocyclic group containing 5 to 12 atoms and 1, 2, 3 or 4 heteroatoms selected from N, O and S.

24. The compound of any of claims 1 to 23 wherein W is an optionally substituted C₁₋₁₀alkyl, C₁₋₁₀alkenyl, C₁₋₁₀alkynyl, C₆₋₁₂aryl or a heterocyclic group containing 5 to 12 atoms and 1, 2, 3 or 4 heteroatoms selected from N, O and S.

25. The compound of any of claims 1 to 24 wherein W comprises an optionally substituted C₆₋₁₂aryl or a heterocyclic group containing 5 to 12 atoms and 1, 2, 3 or 4 heteroatoms selected from N, O and S.

26. The compound of any of claims 1 to 25 wherein W is an optionally substituted C₆₋₁₂aryl or a heterocyclic group containing 5 to 12 atoms and 1, 2, 3 or 4 heteroatoms selected from N, O and S.

27. The compound of any of claims 1 to 25 wherein W comprises an optionally substituted phenyl,

28. The compound of any of claims 1 to 27 wherein W is an optionally substituted phenyl.

29. The compound of any of claims 1 to 25 or 27 wherein W is a benzyl group optionally substituted on the phenyl ring.

30. A compound of any of claims 1 to 29 having formula (III):

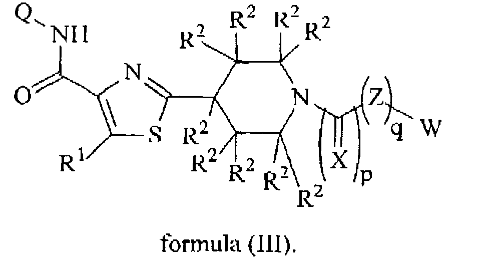

31. A compound of claim 1 selected from:
*N*-[5-(aminocarbonyl)-2-methoxyphenyl]-2-{1-[(4,7-dimethylpyrazolo[5,1-*c*][1,2,4]triazin-3-yl)carbonyl]-4-piperidinyl}-1,3-thiazole-4-carboxamide;
*N-*[5-(aminocarbonyl)-2-methoxyphenyl]-2-[1-(1-benzofuran-2-ylcarbonyl)-4-piperidinyl-1,3-thiazole-4-carboxamide;
*N*-[5-(aminocarbonyl)-2-methoxyphenyl]-2-[1-(3-phenyl-2-propynoyl)-4-piperidinyl]-1,3-thiazole-4-carboxamide;
2-(1-{[2-(allylsulfanyl)-3-pyridinyl]carbonyl}-4-piperidinyl)-*N*-[5-(aminocarbonyl)-2-methoxyphenyl]-1,3-thiazole-4-carboxamide;
*N-*[5-(aminocarbonyl)-2-methoxyphenyl]-2-{1-[(2-chlorophenyl)acetyl]-4-piperidinyl}-1,3-thiazole-4-carboxamide;
*N-*[5*-*(aminocarbonyl)-2-methoxyphenyl]-1-2-{1-[(3,4-dimethylphenoxy)acetyl]-4-piperidinyl}-1,3-thiazole-4-carboxamide;
*N*-[5-(aminocarbonyl)-2-methoxyphenyl]-2-[1-({[4-(dimethylamino)phenyl]amino}carbonothioyl)-4-piperidinyl]-1,3-thiazole-4-carboxamide;
or
*N*-[5-(aminocarbonyl)-2-methoxyphenyl]-2-(1-{[4-(2-pyridinyl)-1-piperazinyl]carbonyl}-4-piperidinyl)-1,3-thiazole-4-carboxamide.

32. A compound of claim 1 selected from:

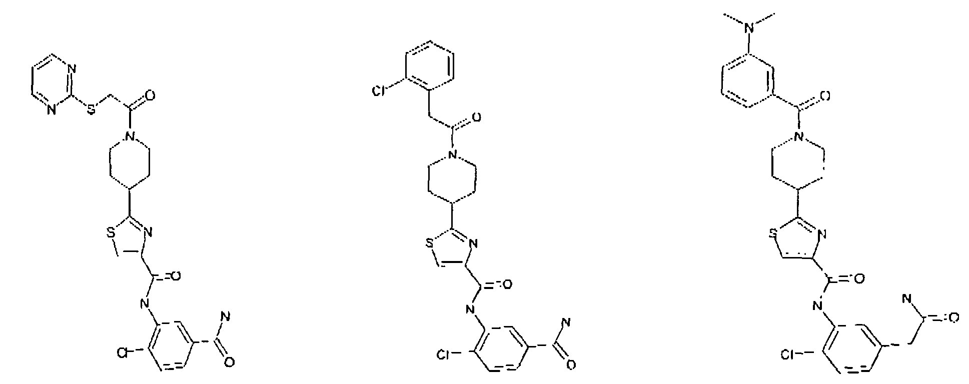

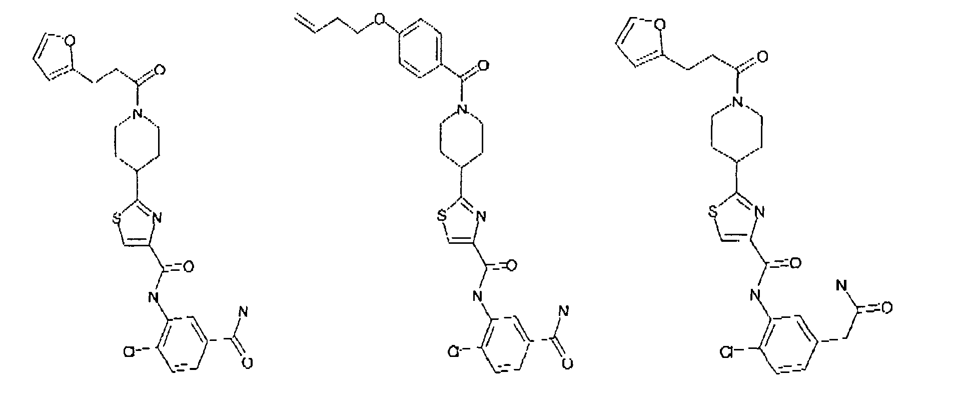

33. The compound of any one of claims 1 to 32 For use in a method of treatment of disease.

34. The compound of any one or claims 1 to 32 for use in therapy or diagnosis.

35. Use of a compound of any one of claims 1 to 34 for the manufacture of a medicament for treating a VEGF-mediated disorder.

36. The use of claim 35, wherein the condition is endometriosis or cancer.

37. A pharmaceutical composition comprising a compound of any one of claims 1-34 in combination with a pharmaceutically acceptable diluent.

38. The use of a VEGF inhibitor which is a compound of any of claims 1-34 for the manufacture of a medicament for treating acute macular degenerative disorder.

39. A VEGF inhibitor which is a compound of any of claims 1-34 for topical administration for the treatment of acute macular degenerative disorder.

40. A topical system for the treatment of acute macular degenerative disorder comprising a VEGF inhibitor which is a compound of any of claims 1-34.

## Patentansprüche

1. Verbindung der Formel (I) oder Formel (II) : 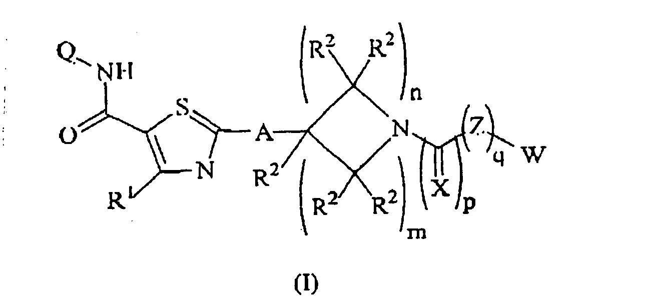 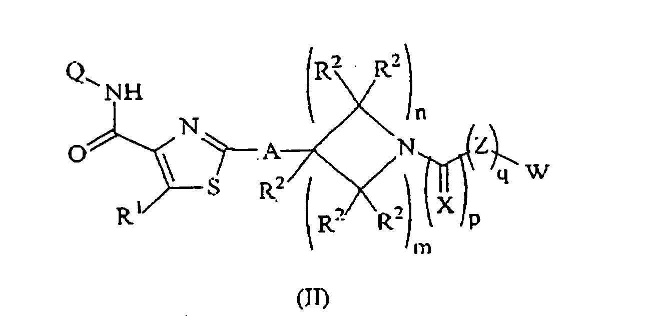 wobei
Q eine gegebenenfalls substituierte C₁₋₁₀-Alkyl-, C₁₋₁₀-Alkenyl-, C₃-₁₂-Cycloalkyl-, C₆-₁₂-Aryl-, C₆₋₁₂-Aryl-C₁₋₁₀-alkyl-, C₁₋₁₀-Alkoxy-, C₆₋₁₂-Aryloxy-, C₆₋₁₂-Aryl-C₁₋₁₀-alkyloxy-, C₁₋₁₀-Alkylthio-, C₆₋₁₂-Aryl-C₁₋₁₀-alkylthio-, Amino-, C₁₋₁₀-Amino-, Di-C₁₋₁₀-alkylamino-, Carboxyl-, Carboxyl-C₁₋₁₀-alkyl-, veresterte Carboxyl-, C₁₋₁₀-Alkylsulfoxyl-, C₁₋₁₀-Alkylsulfonyl-, Nitro-, Carbonitril-, Carbo-C₁₋₁₀-alkoxy, Carbo-C₆₋₁₂-aryloxy- oder eine heterocyclische Gruppe, welche 5 bis 12 Atome und 1, 2, 3 oder 4 Heteroatome, ausgewählt aus N, O und S, enthält, ist, A eine Einfachbindung, cyclisches C₃₋₁₂-Alkylen oder acyclisches C₁₋₁₆-Alkylen ist,
X O oder S ist,
Z O, S oder NR³ ist,
p 0 oder 1 ist,
q 0 oder 1 ist,
n eine ganze Zahl von 0 bis 10 ist,
m eine ganze Zahl von 0 bis 10 ist,
W eine gegebenenfalls substituierte C₁₋₁₀-Alkyl-, C₁₋₁₀-Alkenyl-, C₃₋₁₂-Cycloalkyl-, C₆₋₁₂-Aryl-, C₆₋₁₂-Aryl-C₁₋₁₀-alkyl-, C₁₋₁₀-Alkoxy-, C₆₋₁₂-Aryloxy-, C₆₋₁₂-Aryl-C₁₋₁₀-alkyloxy-, C₁₋₁₀-Alkylthio-, C₆₋₁₂-Aryl-C₁₋₁₀-alkylthio-, Amino-, C₁₋₁₀-Amino-, Di-C₁₋₁₀-alkylamino-, Carboxyl-, Carboxyl-C₁₋₁₀-alkyl-, veresterte Carboxyl-, C₁₋₁₀-Alkylsulfoxyl-, C₁₋₁₀-Alkylsulfonyl-, Nitro-, Carbonitril-, Carbo-C₁₋₁₀-alkoxy, Carbo-C₆₋₁₂-aryloxy- oder eine heterocyclische Gruppe, welche 5 bis 12 Atome und 1, 2, 3 oder 4 Heteroatome, ausgewählt aus N, O und S, enthält, ist,
R¹ H oder C₁₋₁₀-Alkyl ist,
R² unabhängig H oder C₁₋₁₀-Alkyl ist und
R³ H oder C₁₋₁₀-Alkyl ist,
oder ein pharmazeutisch verträgliches Derivat davon,
wobei, wenn eine Gruppe substituiert ist, diese mit einem Halogenatom oder einer Halogenmethylgruppe, wie CF₃ oder CCl₃, einer Sauerstoff-haltigen Gruppe, wie Oxo, Hydroxy, Carboxy, Carboxyalkyl, C₁₋₁₀-Alkoxy, C₁-₁₀-Alkoyl, C₁-₁₀-Alkoyloxy, C₆₋₁₂-Aryloxy, C₆₋₁₂-Aryloxy oder C₆₋₁₂-Aryloyloxy, einer Stickstoff-haltigen Gruppe, wie Amino, Amido, Alkylamino, Dialkylamino, Cyano, Azid, Nitrat, Nitro, -C(O)-NH₂, -C(O)NHR⁴ oder -C(O)NR⁴₂, einer Schwefelhaltigen Gruppe, wie Thiol, C₁₋₁₀-Alkylthiol, Sulfonyl-, Sulfoxid-, -S(O)₂H, -S(O)₂R⁴,
-S(O)-H oder -S(O)-R⁴, einer heterocyclischen Gruppe, welche 5 bis 12 Atome und 1, 2, 3 oder 4 Heteroatome, ausgewählt aus N, O und S, enthält, wie Thienyl, Furanyl, Pyrrolyl, Imidazolyl, Pyrazolyl, Thiazolyl, Isothiazolyl, Oxazolyl, Pyrrolidinyl, Pyrrolinyl, lmidazolidinyl, Imidazolinyl, Pyrazolidinyl, Tetrahydrofuranyl, Pyranyl, Pyronyl, Pyridyl, Pyrazinyl, Pyridazinyl, Benzofuranyl, Isobenzofuryl, Indolyl, Oxyindolyl, lsoindolyl, Indazolyl, Indolinyl, 7-Azaindolyl, lsoindazolyl, Benzopyranyl, Cumarinyl, lsocumarinyl, Chinolyl, lsochinolyl, Naphthridinyl, Cinnolinyl, Chinazolinyl, Pyridopyridyl, Benzoxazinyl, Chinoxadinyl, Chromenyl, Chromanyl, Isochromanyl, Carbolinyl, Oxadiazolyl, Thiadiazolyl, Triazolyl, Tetrazolyl oder Pyrimidinyl, einer C₁₋₁₀-Alkylgruppe, welche selbst substituiert sein kann, oder einer C₆₋₁₂-Arylgruppe, welche selbst substituiert sein kann, wie Phenyl oder substituiertes Phenyl, substituiert sein kann,
wobei R⁴ unabhängig gegebenenfalls substituiertes C₁₋₁₀-Alkyl oder C₆₋₁₂-Aryl ist.

2. Verbindung nach Anspruch 1 der Formel (II).

3. Verbindung nach Anspruch 1 oder Anspruch 2, wobei A eine Einfachbindung ist.

4. Verbindung nach einem der Ansprüche 1 bis 3, wobei X O ist.

5. Verbindung nach einem der Ansprüche 1 bis 3, wobei X S ist und Z NR³ ist.

6. Verbindung nach einem der Ansprüche 1 bis 5, wobei R³ H ist.

7. Verbindung nach einem der Ansprüche 1 bis 6, wobei p = 1.

8. Verbindung nach einem der Ansprüche 1 bis 7, wobei q = 0.

9. Verbindung nach einem der Ansprüche 1 bis 8, wobei die Summe n + m eine ganze Zahl von 2 bis 10 ist.

10. Verbindung nach einem der Ansprüche 1 bis 9, wobei die Summe n + m 4 ist.

11. Verbindung nach einem der Ansprüche 1 bis 10, wobei n = 2 und m = 2.

12. Verbindung nach einem der Ansprüche 1 bis 11, wobei R² H ist.

13. Verbindung nach einem der Ansprüche 1 bis 12, wobei R² H ist.

14. Verbindung nach einem der Ansprüche 1 bis 13, wobei Q eine gegebenenfalls substituierte C₁₋₁₀-Alkyl-, C₁₋₁₀-Alkenyl-, C₃₋₁₂-Cycloalkyl-, C₆₋₁₂-Aryl-, C₆₋₁₂-Aryl-C₁₋₁₀-alkyl-, Carboxyl-, Carboxyl-C₁₋₁₀-alkyl-, veresterte Carboxyl-, C₁₋₁₀-Alkylsulfonyl- oder eine heterocyclische Gruppe, welche 5 bis 12 Atome und 1, 2, 3 oder 4 Heteroatome, ausgewählt aus N, O und S, enthält, ist.

15. Verbindung nach einem der Ansprüche 1 bis 14, wobei Q eine gegebenenfalls substituierte C₆₋₁₂-Aryl oder eine heterocyclische Gruppe, welche 5 bis 12 Atome und 1, 2, 3 oder 4 Heteroatome, ausgewählt aus N, O und S, enthält, umfasst.

16. Verbindung nach einem der Ansprüche 1 bis 15, wobei Q eine gegebenenfalls substituierte C₆₋₁₂-Aryl oder eine heterocyclische Gruppe, welche 5 bis 12 Atome und 1, 2, 3 oder 4 Heteroatome, ausgewählt aus N, O und S, enthält, ist.

17. Verbindung nach einem der Ansprüche 1 bis 15, wobei Q ein gegebenenfalls substituiertes Phenyl umfasst.

18. Verbindung nach einem der Ansprüche 1 bis 17, wobei Q ein gegebenenfalls substituiertes Phenyl ist.

19. Verbindung nach einem der Ansprüche 1 bis 16, wobei Q ein gegebenenfalls substituiertes Furanyl ist.

20. Verbindung nach einem der Ansprüche 1 bis 16, wobei Q ein gegebenenfalls substituiertes Thienyl ist.

21. Verbindung nach einem der Ansprüche 1 bis 16, wobei Q ein Rest ist, welcher aus den in Tabelle 1 dargestellten Resten ausgewählt ist.
**Tabelle 1**
| **Q** | **Rest** |
|---|---|
| Q¹ | 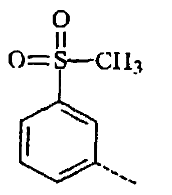 |
| Q² | 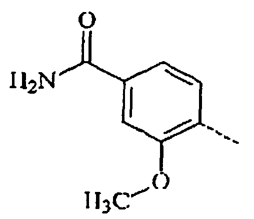 |
| Q³ | 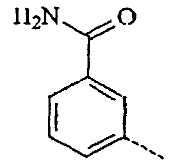 |
| Q⁴ | 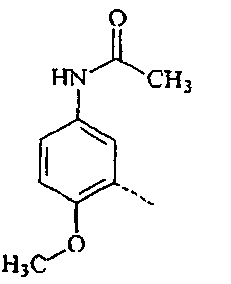 |
| Q⁵ | 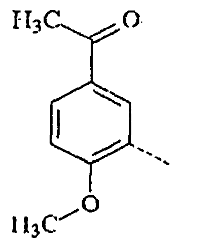 |
| Q⁶ | 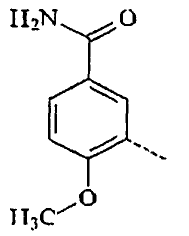 |
| Q⁷ | 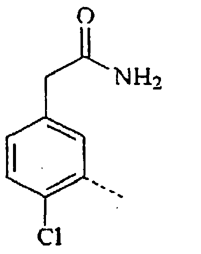 |
| Q⁸ | 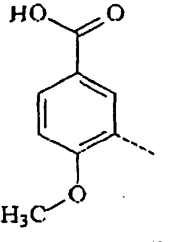 |
| Q⁹ | 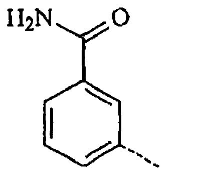 |
| Q¹⁰ | 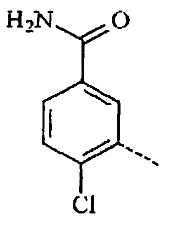 |
| Q¹¹ | 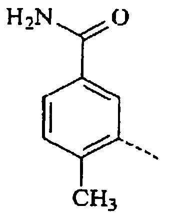 |
| Q¹² | 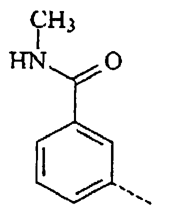 |
| Q¹³ | 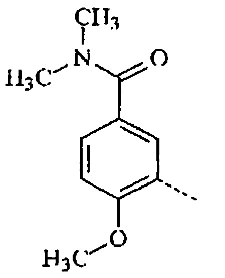 |
| Q¹⁴ | 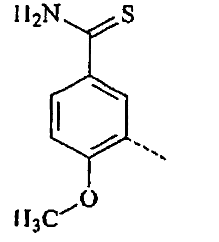 |
| Q¹⁵ | 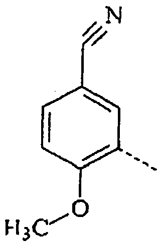 |
| Q¹⁶ | 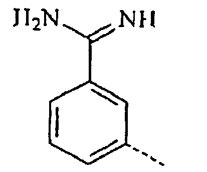 |
| Q¹⁷ | 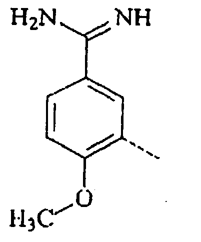 |
| Q¹⁸ | 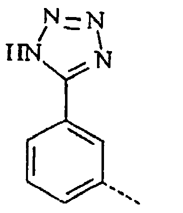 |
| Q¹⁹ | 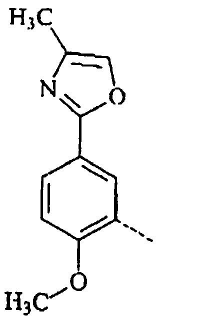 |
| Q²⁰ | 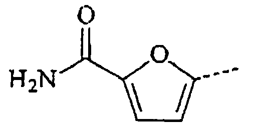 |
| Q²¹ | 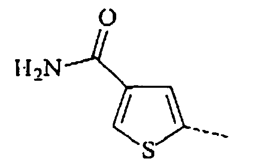 |
| Q²² | 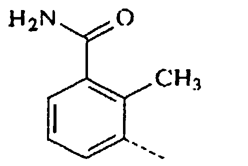 |
| Q²³ | 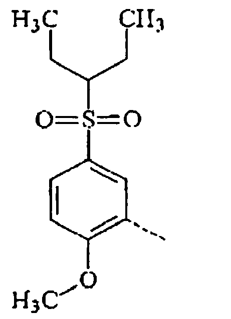 |
| Q²⁴ | 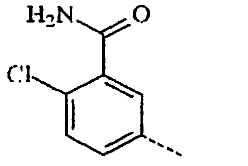 |
| Q²⁵ | 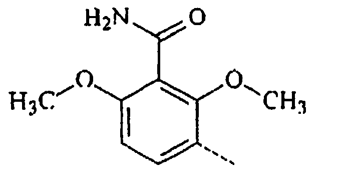 |
| Q²⁶ | 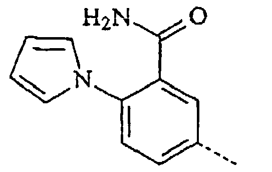 |

22. Verbindung nach Anspruch 21, wobei Q Q₆, Q₇ oder Q₁₀ ist.

23. Verbindung nach einem der Ansprüche 1 bis 22, wobei W eine gegebenenfalls substituierte C₁₋₁₀-Alkyl-, C₁₋₁₀-Alkenyl-, C₃₋₁₂-Cycloalkyl-, C₆₋₁₂-Aryl-, C₆₋₁₂-Aryl-C₁₋₁₀-alkyl-, C₁₋₁₀-Alkoxy-, C₆₋₁₂-Aryloxy-, C₆₋₁₂-Aryl-C₁₋₁₀-alkyloxy-, C₁₋₁₀-Alkylthio-, C₆₋₁₂-Aryl-C₁₋₁₀-alkylthio-, Carboxyl-, Carboxyl-C₁₋₁₀-alkyl-, veresterte Carboxyl-, C₁₋₁₀-Alkylsulfonyl-, Carbo-C₁₋₁₀-alkoxy, Carbo-C₆₋₁₂-aryloxy- oder eine heterocyclische Gruppe, welche 5 bis 12 Atome und 1, 2, 3 oder 4 Heteroatome, ausgewählt aus N, O und S, enthält, ist.

24. Verbindung nach einem der Ansprüche 1 bis 23, wobei W eine gegebenenfalls substituierte C₁-₁₀-Alkyl-, C₁₋₁₀-Alkenyl-, C₁₋₁₀-Alkinyl-, C₆₋₁₂-Aryl- oder eine heterocyclische Gruppe, welche 5 bis 12 Atome und 1, 2, 3 oder 4 Heteroatome, ausgewählt aus N, O und S, enthält, ist.

25. Verbindung nach einem der Ansprüche 1 bis 24, wobei W eine gegebenenfalls substituierte C₆₋₁₂-Aryl- oder eine heterocyclische Gruppe, welche 5 bis 12 Atome und 1, 2, 3 oder 4 Heteroatome, ausgewählt aus N, O und S, enthält, umfasst.

26. Verbindung nach einem der Ansprüche 1 bis 25, wobei W eine gegebenenfalls substituierte C₆₋₁₂-Aryl- oder eine heterocyclische Gruppe, welche 5 bis 12 Atome und 1, 2, 3 oder 4 Heteroatome, ausgewählt aus N, O und S, enthält, ist.

27. Verbindung nach einem der Ansprüche 1 bis 25, wobei W ein gegebenenfalls substituiertes Phenyl umfasst.

28. Verbindung nach einem der Ansprüche 1 bis 27, wobei W ein gegebenenfalls substituiertes Phenyl ist.

29. Verbindung nach einem der Ansprüche 1 bis 25 oder 27, wobei W eine Benzylgruppe ist, welche gegebenenfalls an dem Phenylring substituiert ist.

30. Verbindung nach einem der Ansprüche 1 bis 29 mit der Formel (lll):

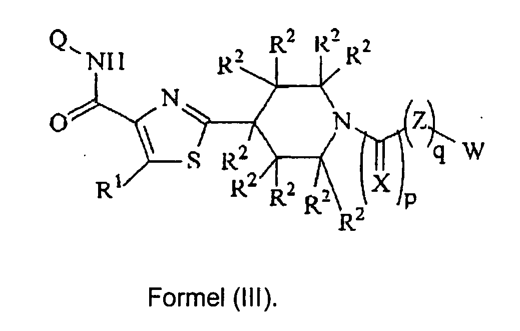

31. Verbindung nach Anspruch 1, ausgewählt aus:
*N*-[5-(Aminocarbonyl)-2-methoxyphenyl]-2-{1-[(4,7-dimethylpyrazolo[5,1-*c*][1,2,4]triazin-3-yl)carbonyl]-4-piperidinyl}-1,3-thiazol-4-carboxamid,
*N*-[5-(Aminocarbonyl)-2-methoxyphenyl]-2-[1-(1-benzofuran-2-ylcarbonyl)-4-piperidinyl]-1,3-thiazol-4-carboxamid,
*N*-[5-(Aminocarbonyl)-2-methoxyphenyl]-2-[1-(3-phenyl-2-propinoyl)-4-piperidinyl]-1,3-thiazol-4-carboxamid,
2-(1-{[2-Allylsulfanyl)-3-pyridinyl]carbonyl}-4-piperidinyl)-*N*-[5-aminocarbonyl)-2-methoxyphenyl]-1,3-thiazol-4-carboxamid,
*N*-[5-(Aminocarbonyl)-2-methoxyphenyl]-2-{1-[(2-chlorphenyl)acetyl]-4-piperidinyl}-1,3-thiazol-4-carboxamid,
*N*-[5-(Aminocarbonyl)-2-methoxyphenyl]-2-(1-[(3 ,4-d imethylphenoxy)acetyl]-4-piperidinyl}-1,3-thiazol-4-carboxamid,
*N*-[5-(Aminocarbonyl)-2-methoxyphenyl]-2-[1-({[4-(dimethylamino)phenyl]amino}carbonothionyl)-4-piperidinyl]-1,3-thiazol-4-carboxamid
oder
*N*-[5-(Aminocarbonyl)-2-methoxyphenyl]-2-(1-{[4-(2-pyrid inyl)-1-piperazinyl]carbonyl}-4-piperidinyl)-1,3-thiazol-4-carboxamid.

32. Verbindung der Formel 1, ausgewählt aus:

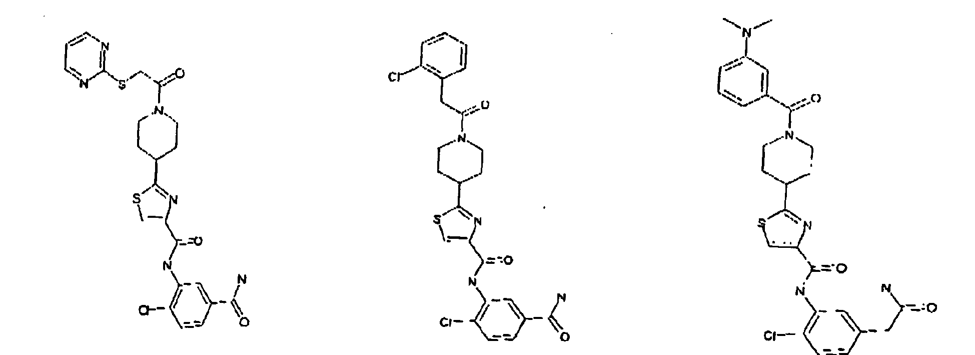

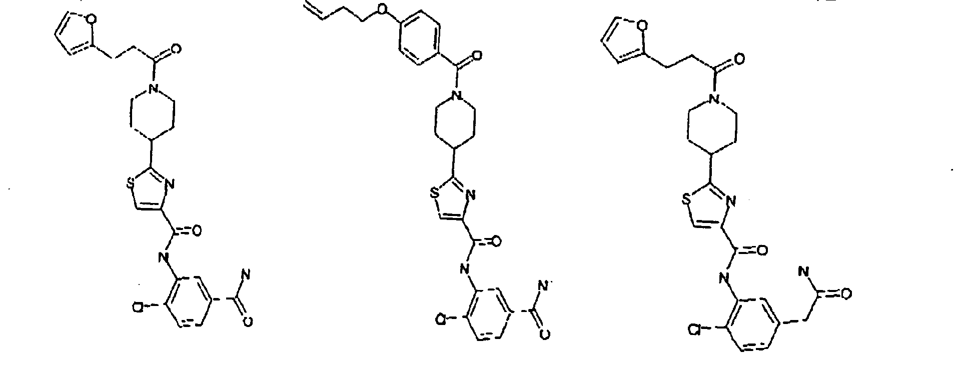

33. Verbindung nach einem der Ansprüche 1 bis 32 zur Verwendung in einem Verfahren zur Krankheitsbehandlung.

34. Verbindung nach einem der Ansprüche 1 bis 32 zur Verwendung in Therapie oder Diagnose.

35. Verwendung einer Verbindung nach einem der Ansprüche 1 bis 34 für die Herstellung eines Medikaments zur Behandlung einer VEGF-vermittelten Erkrankung.

36. Verwendung nach Anspruch 35, wobei der Zustand Endometriose oder Krebs ist.

37. Pharmazeutische Zusammensetzung, welche eine Verbindung nach einem der Ansprüche 1 bis 34 in Verbindung mit einem pharmazeutisch verträglichen Verdünnungsmittel umfasst.

38. Verwendung eines VEGF-Inhibitors, welcher eine Verbindung nach einem der Ansprüche 1 bis 34 ist, für die Herstellung eines Medikaments zur Behandlung von akuter makuladegenerativer Erkrankung.

39. VEGF-Inhibitor, welcher eine Verbindung nach einem der Ansprüche 1 bis 34 ist, zur topischen Verabreichung für die Behandlung von akuter makuladegenerativer Erkrankung.

40. Topisches System zur Behandlung von akuter makuladegenerativer Erkrankung, welches einen VEGF-Inhibitor umfasst, der eine Verbindung nach einem der Ansprüche 1 bis 34 ist.

## Revendications

1. Composé de formule (1) ou de formule (II): 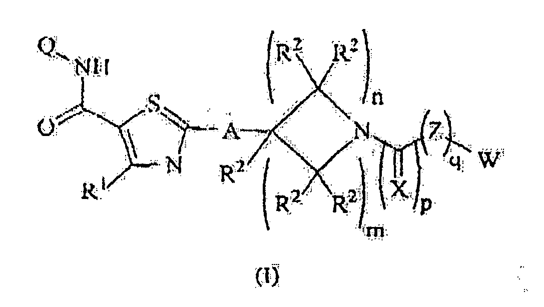 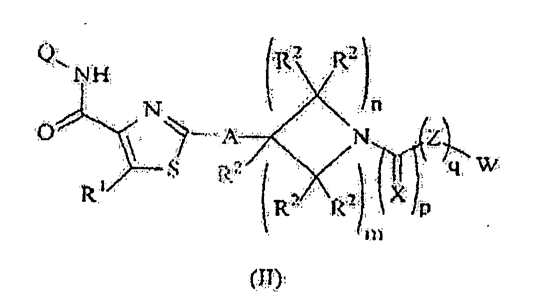 dans lesquelles:
Q est un groupe alkyle en C₁₋₁₀ éventuellement substitué, alcényle en C₁₋₁₀, cycloalkyle en C₃₋₁₂, aryle en C₆₋₁₂, aryl(en C₆₋₁₂)alkyle en C₁₋₁₀, alcoxy en C₁₋₁₀, aryloxy en C₆₋₁₂, aryl(en C₆₋₁₂)alkyloxy en C₁₋₁₀, alkylthio en C₁₋₁₀, aryl(en C₆₋₁₂)alkylthio en C₁₋₁₀, amino, amino en C₁₋₁₀, di(alkylamino en C₁₋₁₀), carboxyle, carboxy(alkyle en C₁₋₁₀), carboxyle estérifié, alkyl(en C₁₋₁₀) sulfoxyle, alkyl(en C₁₋₁₀)sulfonyl, nitro, carbonitrile, carbo(alcoxy en C₁₋₁₀), carbo(aryloxy en C₆₋₁₂), ou un groupe hétérocyclique contenant 5 à 12 atomes et 1, 2, 3 ou 4 hétéroatomes choisis parmi N, O et S;
A est une liaison simple, un groupe alkylène en C₃₋₁₂ cyclique ou alkylène en C₁₋₁₀ acyclique;
X représente O ou S;
X représente O, S ou NR³;
p vaut 0 ou 1;
q vaut 0 ou 1;
n est un nombre entier de 0 à 10;
m est un nombre entier de 0 à 10;
W est un groupe alkyle en C₁₋₁₀ éventuellement substitué, alcényle en C₁₋₁₀, cycloalkyle en C₃₋₁₂, aryle en C₆₋₁₂, aryl(en C₆₋₁₂)alkyle en C₁₋₁₀, alcoxy en C₁₋₁₀, aryloxy en C₆₋₁₂, aryl(en C₆₋₁₂)alkyloxy en C₁₋₁₀, alkylthio en C₁₋₁₀, aryl(en C₆₋₁₂)alkylthio en C₁₋₁₀, amino, amino en C₁₋₁₀, di(alkylamino en C₁₋₁₀), carboxyle, carboxy(alkyle en C₁₋₁₀), carboxyle estérifié, alkyl(en C₁₋₁₀) sulfoxyle, alkyl(en C₁₋₁₀)sulfonyl, nitro, carbonitrile, carbo(alcoxy en C₁₋₁₀), carbo(aryloxy en C₆₋₁₂), ou un groupe hétérocyclique contenant 5 à 12 atomes et 1, 2, 3 ou 4 hétéroatomes choisis parmi N, O et S;
R¹ représente H ou un groupe alkyle en C₁₋₁₀;
R² représente indépendamment H ou un groupe alkyle en C₁₋₁₀; et
R³ représente H ou un groupe alkyle en C₁₋₁₀;
ou l'un de ses dérivés pharmaceutiquement acceptables,
où, lorsqu'un groupe est substitué, il peut être substitué par un atome d'halogène ou un groupe halogénométhyle tel que CF₃ ou CCl₄; un groupe oxygéné tel que oxo, hydroxy, carboxy, carboxyalkyle, alcoxy en C₁₋₁₀, alcoyle en C₁₋₁₀, alcoyloxy en C₁₋₁₀, aryloxy en C₆₋₁₂, aryloyle en C₆₋₁₂ ou aryloyloxy en C₆₋₁₂ ; un groupe azoté tel que amino, amido, alkylamino, dialkylamino, cyano, azido, nitrato, nitro, -C(O)-NH₂, -C(O)NHR⁴ ou -C(O)NR⁴₂ ; un groupe soufré tel que thiol, alkylthiol en C₁₋₁₀, sulfonyle, sulfoxyde, -S(O)₂H₂, -S(O)₂R⁴, -S(O)-H ou -S(O)-R⁴; un groupe hétérocyclique contenant 5 à 12 atomes et 1, 2, 3 ou 4 hétéroatomes choisis parmi N, O et S, tel que thiényle, furanyle, pyrrolyle, imidazolyle, pyrazolyle, thiazolyle, isothiazolyle, oxazolyle ,pyrrolidinyle, pyrrolinyle, imidazolidinyle, imidazolinyle, pyrazolidinyle, tétrahydrofuranyle, pyranyle, pyronyle, pyridyle, pyrazinyle, pyridazinyle, benzofuranyle, isobenzofuryle, indolyle, oxyindolyle, isoindolyle, indazolyle, indolinyle, 7-azaindolyle, isoindazolyle, benzopyranyle, coumarinyle, isocoumarinyle, quinoléyle, isoquinoléyle, naphtridinyle, cinnolinyle, quinazolinyle, pyridopyridyle, benzoxazinyle, quinoxadinyle, chroményle, chromanyle, isochromanyle, carbolinyle, oxadiazolyle, thiadiazolyle, triazolyle, tétrazolyle ou pyrimidinyle; un groupe alkyle en C₁₋₁₀, qui peut être lui-même substitué; ou un groupe aryle en C₆₋₁₂, qui peut être lui-même substitué, tel que phényle ou phényle substitué,
où R⁴ est indépendamment un groupe alkyle en C₁₋₁₀ ou aryle en C₆₋₁₂ éventuellement substitué.

2. Composé selon la revendication 1 de formule (II).

3. Composé selon la revendication 1 ou la revendication 2, où A est une liaison simple.

4. Composé selon l'une quelconque des revendications 1 à 3, où X représente O.

5. Composé selon l'une quelconque des revendications 1 à 3, où X représente S et Z représente NR³.

6. Composé selon l'une quelconque des revendications 1 à 5, où R³ représente H.

7. Composé selon l'une quelconque des revendications 1 à 6, où p= 1.

8. Composé selon l'une quelconque des revendications 1 à 7, où q=0.

9. Composé selon l'une quelconque des revendications 1 à 8, où la somme de n+m est un nombre entier de 2 à 10.

10. Composé selon l'une quelconque des revendications 1 à 9, où la somme de n+m est égale à 4.

11. Composé selon l'une quelconque des revendications 1 à 10, où n=2 et m = 2.

12. Composé selon l'une quelconque des revendications 1 à 11, où R¹ représente H.

13. Composé selon l'une quelconque des revendications 1 à 12, où R² représente H.

14. Composé selon l'une quelconque des revendications 1 à 13, où Q est un groupe alkyle en C₁₋₁₀ éventuellement substitué, alcényle en C₁₋₁₀, cycloalkyle en C₃₋₁₂, aryle en C₆₋₁₂, aryl(en C₆₋₁₂)alkyle en C₁₋₁₀, carboxyle, carboxy(alkyle en C₁₋₁₀), carboxyle estérifié, alkylsulfonyle en C₁₋₁₀ ou un groupe hétérocyclique contenant 5 à 12 atomes et 1, 2, 3 ou 4 hétéroatomes choisis parmi N, O et S.

15. Composé selon l'une quelconque des revendications 1 à 14, où Q comprend un groupe aryle C₆₋₁₂ éventuellement substitué ou un groupe hétérocyclique contenant 5 à 12 atomes et 1, 2, 3 ou 4-hétéroatomes choisis parmi N, O et S.

16. Composé selon l'une quelconque des revendications 1 à 15, où Q est un groupe aryle C₆₋₁₂ éventuellement substitué ou un groupe hétérocyclique contenant 5 à 12 atomes et 1, 2, 3 ou 4-hétéroatomes choisis parmi N, O et S.

17. Composé selon l'une quelconque des revendications 1 à 15, où Q comprend un phényle éventuellement substitué.

18. Composé selon l'une quelconque des revendications 1 à 17, où Q est un phényle éventuellement substitué.

19. Composé selon l'une quelconque des revendications 1 à 16, où Q est un furanyle éventuellement substitué.

20. Composé selon l'une quelconque des revendications 1 à 16, où Q est un thiényle éventuellement substitué.

21. Composé selon l'une quelconque des revendications 1 à 16, où Q est un radical choisi parmi les radicaux présentés dans le tableau 1.
**Tableau 1**
| **Q** | **Radical** |
|---|---|
| Q¹ | 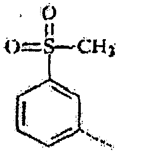 |
| Q² | 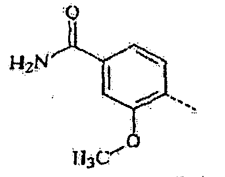 |
| Q³ | 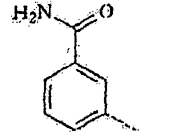 |
| Q⁴ | 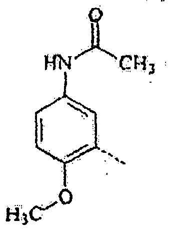 |
| Q⁵ | 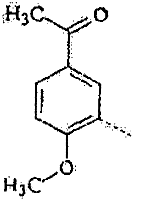 |
| Q⁶ | 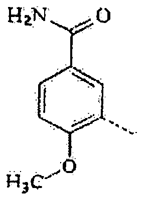 |
| Q⁷ | 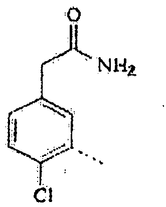 |
| Q⁸ | 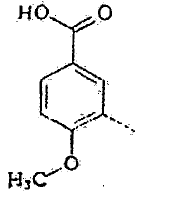 |
| Q⁹ | 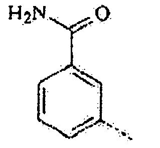 |
| Q¹⁰ | 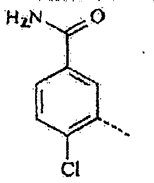 |
| Q¹¹ | 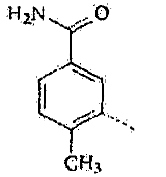 |
| Q¹² | 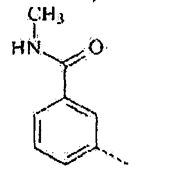 |
| Q¹³ | 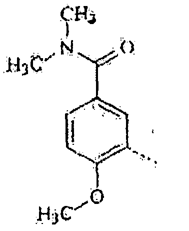 |
| Q¹⁴ | 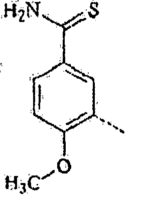 |
| Q¹⁵ | 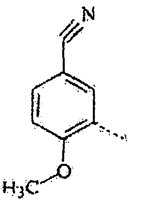 |
| Q¹⁶ | 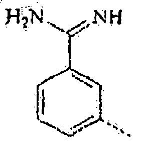 |
| Q¹⁷ | 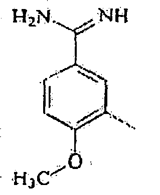 |
| Q¹⁸ | 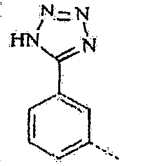 |
| Q¹⁹ | 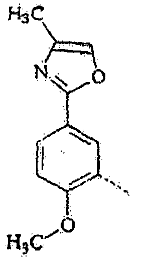 |
| Q²⁰ | 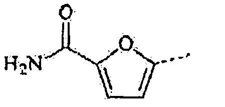 |
| Q²¹ | 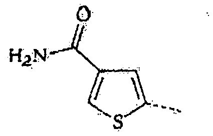 |
| Q²² | 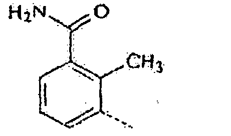 |
| Q²³ | 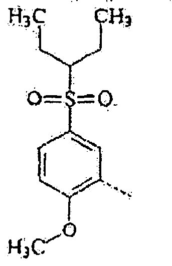 |
| Q²⁴ | 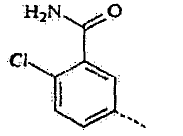 |
| Q²⁵ | 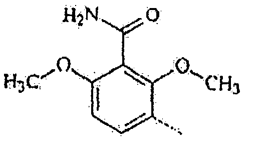 |
| Q²⁶ | 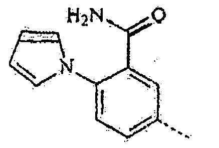 |

22. Composé selon la revendication 21, où Q est Q₆, Q₇ ou Q₁₀.

23. Composé selon l'une quelconque des revendications 1 à 22, où W est un groupe alkyle en C₁₋₁₀ éventuellement substitué, alcényle en C₁₋₁₀, cycloalkyle en C₃₋₁₂, aryle en C₆₋₁₂, aryl(en C₆₋₁₂)alkyle en C₁₋₁₀, alcoxy en C₁₋₁₀, aryloxy en C₆₋₁₂, aryl(en C₆₋₁₂)alkyloxy en C₁₋₁₀, alkylthio en C₁₋₁₀, aryl(en C₆-₁₂)alkylthio en C₁₋₁₀, carboxyle, carboxy(alkyle en C₁₋₁₀), carboxyle estérifié, alkyl(en C₁₋₁₀)sulfonyle, carboxy(alcoxy en C₁₋₁₀), carbo(aryloxy en C₆₋₁₂) ou un groupe hétérocyclique contenant 5 à 12 atomes et l, 2, 3 ou 4 hétéroatomes choisis parmi N, 0 et S.

24. Composé selon l'une quelconque des revendications 1 à 23, où W est un groupe alkyle en C₁₋₁₀ éventuellement substitué, alcényle en C₁₋₁₀, alcynyle en C₁₋₁₀, aryle en C₆₋₁₂ ou un groupe hétérocyclique contenant 5 à 12 atomes et l, 2, 3 ou 4 hétéroatomes choisis parmi N, O et S.

25. Composé selon l'une quelconque des revendications 1 à 24, où W comprend un groupe aryle en C₆₋₁₂ éventuellement substitué ou un groupe hétérocyclique contenant 5 à 12 atomes et 1, 2, 3 ou 4 hétéroatomes choisis parmi N, O et S.

26. Composé selon l'une quelconque des revendications 1 à 25, où W est un groupe aryle en C₆₋₁₂ éventuellement substitué ou un groupe hétérocyclique contenant 5 à 12 atomes et 1, 2, 3 ou 4 hétéroatomes choisis parmi N, O et S.

27. Composé selon l'une quelconque des revendications 1 à 25, où W comprend un phényle éventuellement substitué.

28. Composé selon l'une quelconque des revendications 1 à 27, où W est un phényle éventuellement substitué.

29. Composé selon l'une quelconque des revendications 1 à 25 ou 27, où W est un groupe benzyle éventuellement substitué sur le cycle phényle.

30. Composé selon l'une quelconque des revendications 1 à 29, répondant à la formule (III):

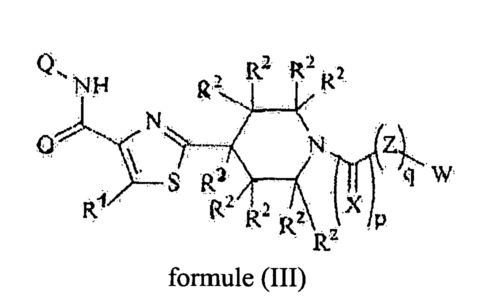

31. Composé selon la revendication 1 choisi parmi:
le *N*-[5-(aminocarbonyl)-2-méthoxyphényl]-2-{1-[(4,7-diméthylpyrazolo[5,1-c}[1,2,4]triazin-3-yl)carbonyl]-4-pipéridinyl} -1,3-thiazole-4-carboxamide;
le *N*-[5-(aminocarbonyl)-2-méthoxyphényl]-2[1-(1-benzofuran-2-ylcarbonyl)-4-pipéridinyl]-1,3-thiazole-4-carboxamide;
le *N*-[5-(anùnocarbonyl)-2-méthoxyphényl]-2-[1-(3-phényl-2-propynoyl)-4-pipéridinyl]-1,3-thiazole-4-carboxamide;
le 2-(1-{[2-(allylsulfanyl)-3-pyridinyl]carbonyl}-4-pipéridinyl)-*N*-[5-(aminocarbonyl)-2-méthoxyphényl]-1,3-thiazole-4-carboxamide;
le *N*-[5-(aminocarbonyl)-2-méthoxyphényl]-2-{1-[(2-chlorophényl)acétyl]-4-pipéridinyl} -1,3-thiazole-4-carboxamide;
le *N*-[5-(aminocarbonyl)-2-méthoxyphenyl]-2-{1-[(3,4-diméthylphénoxy)acétyl]-4-pipéridinyl}-1,3-thiazole-4-carboxamide;
le *N*-[5-(aminocarbonyl)-2-méthoxyphényl]-2-{1-({[4-(diméthylamino)phényl]amino} carbonothiolyl)-4-pipéridinyl]-1,3-thiazole-4-carboxamide;
ou
le *N*-[5-(aminocarbonyl)-2-méthoxyphényl]-2-(1-{[4-(2pyridinyl)-1-pipérazinyl]carbonyl} -4-pipéridinyl)-1,3-thiazole-4-carboxamide.

32. Composé selon la revendication 1 choisi parmi:

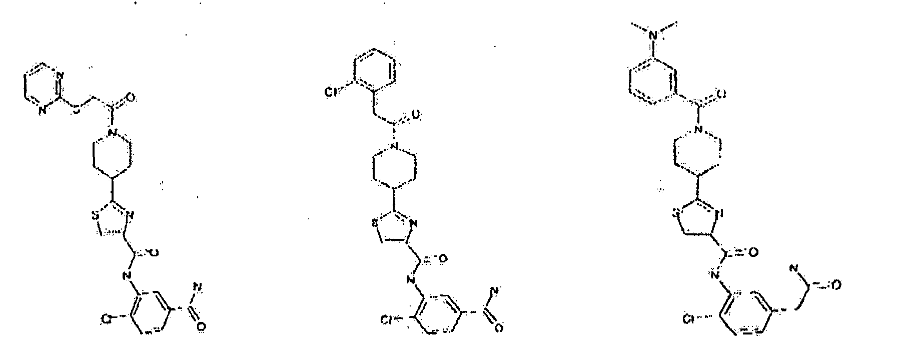

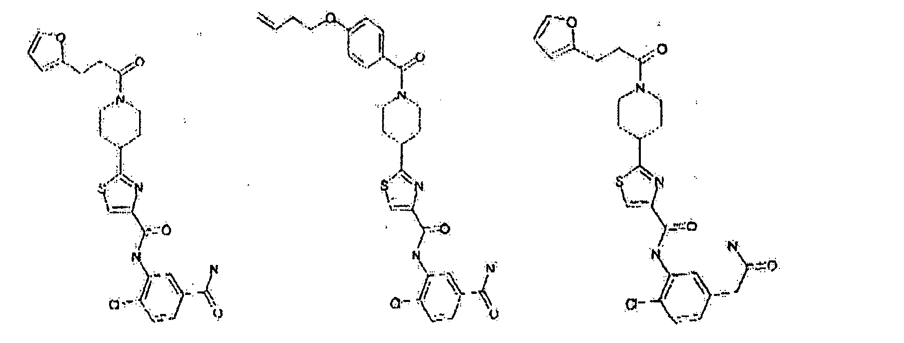

33. Composé selon l'une quelconque des revendications 1 à 32, à utiliser dans un procédé de traitement d'une maladie.

34. Composé selon l'une quelconque des revendications 1 à 32, à utiliser en thérapie ou en diagnostic.

35. Utilisation d'un composé selon l'une quelconque des revendications 1 à 34 pour la fabrication d'un médicament destiné au traitement d'un trouble à médiation de VEGF.

36. Utilisation selon la revendication 35, dans laquelle l'affection est une endométriose ou un cancer.

37. Composition pharmaceutique comprenant un composé selon l'une quelconque des revendications 1 à 34 en combinaison avec un diluant pharmaceutiquement acceptable.

38. Utilisation d'un inhibiteur de VEGF qui est un composé selon l'une quelconque des revendications 1 à 34 pour la fabrication d'un médicament destiné au traitement d'une dégénérescence maculaire aiguë.

39. Inhibiteur de VEGF qui est un composé selon l'une quelconque des revendications 1 à 34 pour l'administration topique destiné au traitement d'une dégénérescence maculaire aiguë.

40. Système topique destiné au traitement d'une dégénérescence maculaire aiguë, comprenant un inhibiteur de VEGF qui est un composé selon l'une quelconque des revendications 1 à 34.
